(19) Europäisches Patentamt
European Patent Office
Office européen des brevets

(11) EP 1 824 986 B1

(12) **FASCICULE DE BREVET EUROPEEN**

(45) Date de publication et mention de la délivrance du brevet:
**06.01.2010 Bulletin 2010/01**

(21) Numéro de dépôt: **05822899.0**

(22) Date de dépôt: **25.11.2005**

(51) Int Cl.:
*C12P 21/00* (2006.01) *C12P 19/00* (2006.01)
*A23J 3/06* (2006.01) *A23L 1/052* (2006.01)
*A23L 1/0562* (2006.01) *A61K 8/04* (2006.01)
*A61K 8/65* (2006.01) *A61K 8/66* (2006.01)
*A61K 8/73* (2006.01) *A61K 9/06* (2006.01)

(86) Numéro de dépôt international:
**PCT/FR2005/002939**

(87) Numéro de publication internationale:
**WO 2006/056700 (01.06.2006 Gazette 2006/22)**

(54) **BIOMATERIAU CAPABLE D'EFFECTUER SUCCESSIVEMENT UNE TRANSITION SOLUTION/GEL PUIS UNE TRANSITION GEL/SOLUTION**

BIOMATERIAL MIT DER FÄHIGKEIT ZUR NACHEINANDER ERFOLGENDEN DURCHFÜHRUNG EINES LÖSUNG/GEL-ÜBERGANGS UND ANSCHLIESSENDEN GEL/LÖSUNG-ÜBERGANGS

BIOMATERIAL WHICH CAN SUCCESSIVELY CARRY OUT A SOLUTION/GEL TRANSITION THEN A GEL/SOLUTION TRANSITION

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **26.11.2004 FR 0452774**

(43) Date de publication de la demande:
**29.08.2007 Bulletin 2007/35**

(73) Titulaire: **Université de Cergy-Pontoise
95011 Cergy-Pontoise Cedex (FR)**

(72) Inventeurs:
- **LARRETA-GARDE, Véronique
F-95290 L'Isle Adam (FR)**
- **GIRAUDIER, Sébastien
F-95800 Cergy (FR)**

(74) Mandataire: **Breese, Pierre
NOVAGRAAF IP
122, rue Edouard Vaillant
92593 Levallois-Perret Cedex (FR)**

(56) Documents cités:
**EP-A- 0 492 406 WO-A-97/40701**

- **BERRY HUGUES ET AL: "Gel-sol transition can describe the proteolysis of extracellular matrix gels" BIOCHIMICA ET BIOPHYSICA ACTA, vol. 1524, no. 2-3, 15 décembre 2000 (2000-12-15), pages 110-117, XP002336320 ISSN: 0006-3002**
- **GIRAUDIER SEBASTIEN ET AL: "Influence of weak and covalent bonds on formation and hydrolysis of gelatin networks" BIOMACROMOLECULES, vol. 5, no. 5, septembre 2004 (2004-09), pages 1662-1666, XP002336321 ISSN: 1525-7797**
- **CRESCENZI VITTORIO ET AL: "New gelatin-based hydrogels via enzymatic networking." BIOMACROMOLECULES, vol. 3, no. 6, 2002, pages 1384-1391, XP002336327 ISSN: 1525-7797**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 824 986 B1

## Description

La présente invention concerne, pour le domaine de la gélification, un biomatériau capable d'effectuer successivement une transition contrôlée solution/gel puis gel/solution et un procédé de préparation d'un tel biomatériau.

Les gels sont utilisés du fait de leurs propriétés particulières dans de nombreux domaines, notamment alimentaire, cosmétique ou pharmaceutique. Un gel est composé d'au moins deux composants dont l'un, très fortement majoritaire, correspond au solvant liquide et l'autre est un composant que l'on peut qualifier de solide dispersé au sein du solvant. À partir d'une solution ou d'une dispersion à l'état liquide, la formation du gel est le résultat d'une agrégation partielle des particules solides. On appelle cette transformation la transition solution/gel.

Il est bien connu de l'art antérieur des compositions pour obtenir des gels « physiques ». Un gel physique correspond à un assemblage macroméculaire constitué par des monomères liés entre eux par des liaisons de faible énergie (Van der Waals, liaisons hydrogènes, polaires, etc.). La stabilité de cet assemblage est associée à une certaine plage de conditions physico-chimiques (pH, concentration en monomères, température, qualité du solvant, force ionique, etc.). En dehors de cette plage, le gel redevient solution. La transition solution/gel est donc réversible pour les gels physiques. Ainsi, la charpente des gels physiques est très sensible à l'environnement physico-chimique et un très faible changement de qualité de solvant peut détruire complètement l'édifice et induire ainsi une transition gel-solution. A l'inverse, l'association polymérique conduisant au gel peut se faire par un faible changement de qualité de solvant.

On connaît également dans l'art antérieur des gels qualifiés de « chimique ». Un gel chimique correspond également à un assemblage macromoléculaire, pour autant les monomères y sont associés par des liaisons de forte énergie (covalentes). La stabilité de cet assemblage est donc très grande. Pour autant, si ces gels chimiques présentent une stabilité améliorée, le seul moyen d'effectuer une transition gel/solution consiste à détruire les liaisons covalentes du polymère. C'est pourquoi, la transition gel/solution de ce type de polymère est dite irréversible.

Une famille de gels chimiques correspond aux gels catalysés enzymatiquement. Ce mode de gélification est surtout observé dans les grands processus biologiques. La coagulation sanguine, la cicatrisation, la formation de peau, l'assemblage des matrices extracellulaires sont des processus biologiques où le passage des protéines solubles à l'état de gel est indispensable et ont en commun une famille d'enzymes : Les transglutaminases (Tgases), lesquelles sont indispensables aux processus de gélification. Cette famille de protéines est ubiquitaire et on la retrouve aussi bien chez les procaryotes que chez les eucaryotes. Il existe ainsi 8 Tgases différentes chez l'homme. Ces enzymes ont la propriété d'incorporer des groupes amines sur des chaînes latérales glutaminyl des protéines. Cette activité permet de lier les protéines de manière covalente. Les Tgases catalysent ainsi la polymérisation des protéines responsables de la formation des réseaux gélifiés biologiques. Les Tgases permettent d'obtenir des gels à partir de nombreuses protéines dans l'industrie alimentaire, et notamment pour la fabrication du surimi ou le durcissement de nombreux dérivés carnés (Jambon, nourriture reconstituée, etc.). On peut citer, à titre d'exemples de protéines polymérisables, la gélatine, la fibrine, la gliadine, la myosine, la globuline (7S et 11S), l'actine, la myoglobine, les protéines du petit lait, notamment les caséines et la lactaglobuline, les protéines du soja, du blé et notamment la gluténine, du jaune et du blanc d'oeuf, et notamment l'ovalbumine.

Un des gels protéiques les plus utilisés est le gel de gélatine. La gélatine est obtenue à partir du collagène qui est une protéine de structure ubiquitaire. Le collagène peut se trouver à l'état soluble sous forme de monomères ou de trimères associés en triples hélices. Ces triples hélices peuvent s'associer en fibrilles qui peuvent s'associer en fibres. La triple hélice de collagène est instable à la température du corps. La gélatine est obtenue par dénaturation du collagène. Les tissus contenant du collagène subissent ainsi un traitement acide ou alcalin, qui aboutit à la dénaturation de la triple hélice du collagène. La possibilité de faire des fibres est alors totalement perdue. Un traitement acide aboutit à la formation de gélatine de type A et un traitement alcalin à une gélatine de type B. La solution de gélatine est donc composée de chaînes isolées de collagène (monomères de collagènes). Les utilisations de la gélatine étant multiples, il est parfois nécessaire de créer des gels de gélatine dans des conditions où les gels physiques n'existent pas (hautes températures, pH extrême ou force ionique particulière). Pour former le réseau nécessaire au gel, les chaînes de gélatine sont alors pontées par des liens covalents et, notamment, par l'action des Tgases. Les gels ainsi obtenus sont des gels chimiques.

Aujourd'hui de nombreux domaines nécessitent l'utilisation de gels chimiques du fait de leur stabilité améliorée. Toutefois, leur « irréversibilité » limite leurs utilisations potentielles dans les domaines cosmétiques, alimentaires ou encore pharmaceutiques. Une plus grande maîtrise des propriétés mécaniques des différents gels chimiques constitue donc un enjeu essentiel pour étendre leur potentialité.

L'analyse du vivant a révélé l'existence de systèmes extrêmement dynamiques. Dans les tissus vivants, les cellules sont en interaction avec une structure appelée la matrice extracellulaire (MEC) qui est riche en protéines. Cette structure est principalement localisée sous les cellules épithéliales et autour des tissus conjonctifs. Les cellules peuvent synthétiser différents composants de la matrice extracellulaire tels que le collagène qui intervient dans l'élasticité ou la fibronectine qui intervient dans les mécanismes de l'adhérence. Parallèlement, la cellule produit également des protéases qui interviennent dans la dégradation de la matrice extracellulaire. La cellule intervient donc simultanément dans la

construction et la dégradation de la matrice extracellulaire. La structure de la matrice extracellulaire n'est donc pas une structure irréversible et statique mais correspond à un équilibre dynamique résultant de la balance entre les activités de construction et de dégradation des protéines synthétisées par la cellule.

De la même manière, les caillots formés en réponse au mécanisme de coagulation constituent également des systèmes dynamiques. Ainsi, par la synthèse d'une cascade de facteurs enzymatiques, on assiste à la formation d'un caillot insoluble qui naît de la formation d'un réseau de protéines solubles. Ce caillot sera ensuite éliminé au cours de la réaction de cicatrisation.

Dans ces équilibres dynamiques, les réseaux de protéines s'associent pour devenir insolubles et former des gels, ce qui peut être assimilé à des transitions solution/gel. Dans le même temps, les réseaux protéiques sont également détruits par l'action de protéases, ce type de transition pouvant cette fois être assimilé à des transitions gel/solution. On assiste ainsi parfois à des transitions successives comme dans la coagulation où le caillot est formé en premier lieu, puis dégradé pour laisser place à d'autres réactions selon des mécanismes mal compris.

La transition solution/gel dans ces processus biologiques est le plus souvent associée à la famille des transglutaminases décrite précédemment. La transition opposée, à savoir gel/solution est de son côté associée à l'activité antagoniste d'enzymes de type protéolytique.

Une des familles les plus étudiées est celle des métalloprotéinases de la matrice (MMP). Elles forment une famille d'endopeptidases dépendantes du zinc qui dégradent la plupart des protéines de la matrice extracellulaire. Il existe toutefois un grand nombre de familles de protéases différentes. On peut citer, à titre d'exemple de familles de protéases, les sérines protéases, telles que la trypsine, la matriptase, les Cystéines et Aspartate protéases, telles que les cathepsines B et L et les cathepsines D et G, et la famille ADAM.

Un grand nombre des enzymes orchestrant ce type de réaction, comme les transglutaminases ou encore les métalloprotéases sont caractérisées par les biochimistes et les enzymologistes. La description de ces enzymes, et notamment des différentes constantes qui leur sont associées, est ainsi bien maîtrisée. Pour autant leur comportement, s'il est connu en solution et de façon isolée, ne l'est pas dans des milieux de type gel et en présence d'une activité antagoniste.

Le travail de modélisation des inventeurs et d'autres équipes a permis de mieux comprendre les paramètres intervenant dans cet équilibre dynamique existant *in vivo.* Les derniers travaux de modélisation et d'expérimentation réalisés par les inventeurs leur ont permis de développer un nouveau modèle de cet équilibre dynamique qui intègre avec plus de justesse les paramètres impliqués dans celui-ci.

De façon surprenante, les modélisations effectuées par les inventeurs ont mis en évidence qu'il était possible d'obtenir « *in vitro* » de tels équilibres dynamiques. Les inventeurs ont ainsi pu obtenir des gels protéiques qui sont capables d'effectuer successivement une transition solution/gel puis gel/solution dans des conditions déterminées, et notamment selon une cinétique déterminée.

En conséquence, un premier objet de l'invention porte sur un biomatériau obtenu par un procédé selon l'une quelconque des revendications 1 à 6 et qui comprend au moins un monomère capable de former des polymères, de préférence par des liaisons de faible énergie, ou un mélange desdits monomères et de leurs polymères, et un premier type d'enzyme capable de dégrader lesdits polymères, le dit biomatériau se présentant soit sous la forme d'un gel, soit sous la forme d'une solution, et étant susceptible d'effectuer successivement une première transition solution/gel puis, sous l'action du premier type d'enzyme, une seconde transition gel/solution.

Un tel biomatériau permet ainsi de développer des produits présentant des propriétés jusqu'alors inconnues dans les domaines cosmétiques, alimentaires ou pharmaceutiques.

Dans le domaine cosmétique, le biomatériau selon l'invention peut ainsi permettre de réaliser de nouveaux cosmétiques, tels que des masques de beauté pouvant adopter la texture d'un gel durant le temps d'application nécessaire avant de repasser à l'état de solution pour permettre une élimination simplifiée et agréable pour l'utilisateur.

Dans le domaine agroalimentaire, le biomatériau selon l'invention peut ainsi permettre de réaliser de nouveaux produits aux textures inconnues, tels que des biscuits ou des friandises fourrés, dont la garniture peut prendre la forme d'un gel durant l'enrobage avant de repasser à l'état de solution plus ou moins visqueuse une fois l'enrobage terminé.

Dans le domaine pharmaceutique ou cosmétique, le biomatériau selon l'invention peut également permettre d'obtenir des gels, emprisonnant un principe actif, capables de relarguer ledit principe actif en repassant à l'état de solution avec une cinétique déterminée. L'utilisation d'enzymes capables de dégrader les protéines (i) polymérisées dans des conditions de température, de pH, de concentrations ioniques spécifiques (calcium, magnésium, ou autre) ou en présence de cofacteurs déterminés peut permettre d'adapter ce relargage à un environnement donné (intestin, estomac, etc.).

Les compositions cosmétiques, pharmaceutiques ou alimentaires peuvent en outre contenir d'autres agents, tels que des agents actifs pour les compositions cosmétiques ou pharmacologiques, ou des agents de formulation pour les compositions alimentaires.

Le monomère compris dans le biomatériau de l'invention est une protéine ou un saccharide, lequel est d'origine naturelle ou synthétique.

Le premier type d'enzyme est présent dans le biomatériau sous une forme non-inactivée. Le premier type d'enzyme capable de dégrader les polymères peut être soit sous une forme active dans le biomatériau, soit sous une forme activable dans des conditions spécifiques, tel qu'un pH donné, en présence d'une espèce ionique ou d'un cofacteur spécifique, de façon à obtenir la transition gel/solution dans des conditions spécifiques, telles que dans un organe particulier (estomac, intestin, cavité buccale, etc.) ou dans un environnement particulier.

Le biomatériau de l'invention comprend en outre un solvant approprié pour permettre la polymérisation du monomère et la dégradation du polymère formé par le premier type d'enzyme. De préférence, le solvant utilisé est un solvant aqueux, tel que de l'eau ou des solutions tampons au pH souhaité (tampon phosphate ou tris).

Selon un premier mode de réalisation particulier du biomatériau de l'invention, ledit biomatériau sous forme de gel a été lyophilisé selon des techniques connues de l'homme du métier. Le biomatériau ne comprend alors pas de solvant. La deuxième transition gel/solution s'effectue alors seulement après que ledit biomatériau ait été mis en contact avec un solvant approprié. Ce mode de réalisation permet ainsi d'associer la durée de vie du gel, après sa fabrication, à sa présence dans un environnement particulier ou dans un organe spécifique (estomac, intestin, cavité buccale, etc.).

Selon un mode de réalisation préféré de l'invention, le biomatériau comprend en outre au moins un second type d'enzyme capable d'effectuer des liaisons covalentes entre lesdits monomères, le dit biomatériau est alors susceptible d'effectuer successivement une première transition solution/gel sous l'action du second type d'enzyme puis une seconde transition gel/solution sous l'action du premier type d'enzyme.

Avantageusement, les deux types d'enzymes sont présents simultanément dans le biomatériau sous une forme non-inactivée.

Avantageusement encore, les enzymes sont réparties de façon homogène dans le biomatériau.

Selon un deuxième mode de réalisation particulier du biomatériau de l'invention, le biomatériau comprend un monomère de nature protéique. La quasi-totalité des protéines sont capables de former des gels physiques du fait de leurs propriétés de polyélectrolytes. Les protéines susceptibles de polymériser pour former des gels physiques et utilisables dans le procédé selon l'invention sont: le collagène et ses dérivés, telle que la gélatine, la fibrine, la gliadine, la myosine, la globuline (7S et 11S), l'actine, la myoglobine, les protéines du petit lait, notamment les caséines et la lactaglobuline, les protéines du soja, du blé et notamment la gluténine, du jaune et du blanc d'oeuf, et notamment l'ovalbumine. De préférence, les protéines polymérisables utilisées sont choisies parmi la fibrine et le collagène et ses dérivés, telles que les gélatine de type A et B. En présence d'enzyme capables d'effectuer des liaisons covalentes entre les monomères protéiques, il est possible d'utiliser des protéines ne polymérisant pas naturellement.

La concentration de protéines polymérisables, sous la forme de monomères ou d'un mélange desdits monomères et de leurs polymères, dans le biomatériau est fonction de la nature de la protéine utilisée. De préférence, la concentration en protéine polymérisable est comprise entre 0,1 et 30% en poids par rapport au poids total du biomatériau, de préférence entre 0,2 et 20%, préférentiellement encore entre 0,5 et 15%, et de manière particulièrement préférée entre 1 et 10%.

De nombreuses enzymes capables de dégrader des polymères protéiques (protéases) sont connues de l'homme du métier. A titre d'exemple de telles enzymes, on peut citer les métalloprotéinase, telle que les métalloprotéinases de la famille de type MMP ou des métalloprotéinases apparentées, telle que la thermolysine, les sérines protéases, telles que la trypsine et la matriptase, les Cystéines et Aspartate protéases, telles que les cathepsines B et L et les cathepsines D et G, et la famille ADAM. De préférence, l'enzyme capable de dégrader des polymères de protéines est une métalloprotéinase, préférentiellement une thermolysine, notamment bactérienne, et de manière particulièrement préférée une thermolysine isolée à partir de *Bacillus thermoproteolyticus rokko.*

La concentration en enzyme capable de dégrader des polymères protéiques dépend du monomère de protéine utilisée et de la concentration de celui-ci. Cette concentration d'enzyme peut être calculée simplement pour un type de monomère protéique donné et à une concentration donnée, selon le protocole décrit dans les exemples qui suivent. Ainsi, dans le cas de la thermolysine de *Bacillus thermoproteolyticus rokko* et pour un gel de gélatine de type A à 5%, la concentration en thermolysine est supérieure ou égale à $10^{-4}$ U/ml, de préférence supérieure ou égale à $10^{-3}$ U/ml.

De nombreuses protéines sont également capables d'effectuer des liaisons covalentes entre des protéines sont connues de l'homme du métier. À titre d'exemple de telles protéines, on peut citer la famille des lysyl oxydases et la famille des transglutaminases telles que la sous-unité A du facteur XIII, les Tgases 1 à 7 de mammifères ou encore les tgases bactériennes. De préférence, l'enzyme capable d'effectuer des liaisons covalentes entre des protéines est une transglutaminase, préférentiellement une transglutaminase bactérienne, et de manière particulièrement préférée une transglutaminase isolée à partir de *Streptoverticillium sp.*

Les concentrations en enzyme capable d'effectuer des liaisons covalentes entre des protéines et en enzyme capable de dégrader des polymères protéiques dépendent d'une part du rapport de la concentration d'enzyme capable d'effectuer des liaisons covalentes entre des protéines sur la concentration en enzyme capable de dégrader des polymères protéiques, et d'autre part de la protéine polymérisable utilisée et de la concentration de celle-ci. Ces concentrations d'enzymes peuvent être calculées simplement selon le protocole décrit dans les exemples.

Le rapport des concentrations enzymatiques et les concentrations enzymatiques utilisées dépendront également du temps de gel (transition solution/gel) et de la durée de vie du gel souhaités.

Avantageusement, le rapport de la concentration d'enzyme capable d'effectuer des liaisons covalentes entre des protéines sur la concentration en enzyme capable de dégrader des polymères protéiques est supérieur ou égal à 1, de préférence supérieur ou égal à 10, et de manière particulièrement préférée supérieur ou égal à 20.

Avantageusement encore, la concentration en enzyme capable d'effectuer des liaisons covalentes entre les protéines est supérieure à 0,001 U/ml, de préférence supérieure à 0,01 U/ml et de manière particulièrement préférée supérieure à 0,1 U/ml.

Avantageusement encore, et dans le cas d'une solution de gélatine de type A à 5% en présence de la transglutaminase de *Streptoverticillium sp* et de la thermolysine de *Bacillus thermoproteolyticus rokko,* le rapport de la concentration de transglutaminase en U/ml sur la concentration en thermolysine en U/ml est supérieure à 75, de préférence supérieure ou égale à 80. En outre, la concentration en transglutaminase est supérieure ou égale à 0,01 U/ml, de préférence supérieure ou égale à 0,1 U/ml et de manière particulièrement préférée supérieure ou égale à 0,4 U/ml.

Selon un troisième mode de réalisation particulier de la présente invention, le biomatériau selon l'invention comprend un monomère de nature saccharidique. Les saccharides utilisables pour le biomatériau selon l'invention sont: les carraghénanes, les alginates, les pectines, le chitosan, la cellulose, la chitine, le glycogène ou encore l'amidon.

La concentration en saccharides polymérisables, sous forme de monomères ou d'un mélange desdits monomères et de leurs polymères, dans le biomatériau est fonction de la nature du saccharide utilisé. De préférence, la concentration en saccharide polymérisable est comprise entre 0,1 et 30% en poids par rapport au poids total du biomatériau, de préférence entre 0,2 et 20%, préférentiellement encore entre 0,5 et 15%, et de manière particulièrement préférée entre 1 et 10%.

De nombreuses enzymes capables de dégrader des polysaccharides sont connues de l'homme du métier. A titre d'exemple de telles enzymes, on peut citer les carraghénases pour les carraghénanes, les pectines lyases, les polygalacturonases et les pectines estérases pour les pectines, les alginates lyases pour les alginates, les cellulases pour la cellulose ou encore la phosphorylase pour le glycogène.

La concentration en enzyme capable de dégrader des polysaccharides dépend du monomère utilisé et de la concentration de celui-ci. Cette concentration d'enzyme peut être calculée simplement pour un type de monomère donné et à une concentration donnée, selon le protocole décrit dans les exemples.

De nombreuses enzymes sont également capables d'effectuer des liaisons covalentes entre des saccharides sont connues de l'homme du métier. À titre d'exemple de telles enzymes, on peut citer la famille des alginates épimérases pour les alginates, des synthases pour la cellulose, ou encore la glycogène synthase pour le glycogène.

Les concentrations en enzyme capable d'effectuer des liaisons covalentes entre des saccharides et en enzyme capable de dégrader des polysaccharides dépend d'une part du rapport de la concentration d'enzyme capable d'effectuer des liaisons covalentes entre des saccharides sur la concentration en enzyme capable de dégrader des polysaccharides et d'autre part du saccharide polymérisable utilisé et de la concentration de celui-ci. Ces concentrations d'enzymes peuvent être calculées simplement selon le protocole décrit dans les exemples.

Comme précédemment, le rapport des concentrations enzymatiques et les concentrations enzymatiques utilisées dépendront également du temps de gel (transition solution/gel) et de la durée de vie du gel souhaités.

Avantageusement, le rapport (concentration d'enzyme capable d'effectuer des liaisons covalentes entre des saccharides)/(concentration en enzyme capable de dégrader des polysaccharides) est supérieur ou égal à 1, de préférence supérieur ou égal à 10, et de manière particulièrement préféré supérieur ou égal à 20.

Avantageusement encore, la concentration en enzyme capable d'effectuer des liaisons covalentes entre des saccharides est supérieure à 0,001 U/ml, de préférence supérieure à 0,01 U/ml et de manière particulièrement préférée supérieure à 0,1 U/ml.

Selon un quatrième mode de réalisation particulier de l'invention, le biomatériau selon l'invention comprend un monomère de nature saccharidique et un monomère de nature protéique. Un tel composé permet d'obtenir un gel présentant des propriétés rhéologiques spécifiques et, potentiellement, un gel présentant une texture recherchée.

Le biomatériau peut également comprendre un ou plusieurs composants additionnels couramment utilisés dans les domaines cosmétiques, pharmaceutiques ou alimentaires.

Un deuxième objet de l'invention porte sur un procédé de préparation d'un biomatériau selon la revendication 1, le biomatériau se présentant soit sous la forme d'un gel, soit sous la forme d'une solution, et étant susceptible d'effectuer successivement une transition solution/gel puis une seconde transition gel/solution, **caractérisé en ce qu'**il comprend le mélange dans un solvant approprié:

(i) d'au moins un monomère capable de former des polymères, de préférence par des liaisons de faible énergie;

(ii) d'un premier type d'enzyme capable de dégrader lesdits polymères.

La concentration en monomères capables de former des polymères, de préférence par des liaisons de faible

énergie, et la concentration en enzyme capable de dégrader lesdits polymères pour obtenir un biomatériau qui présente le temps de gel et la durée de vie de gel souhaités sont déterminées selon le protocole décrit dans les exemples.

À titre d'exemple, un biomatériau comprenant 5% de gélatine de type A, à titre de monomère capable de former des polymères par des liaisons de faible énergie, et de la thermolysine isolée à partir de *Bacillus thermoproteolyticus rokko* et présentant un temps de gel de 68 minutes et une durée de vie du gel de 254 minutes peut être obtenu à 27°C en utilisant 0,0085 U/ml de thermolysine.

Selon un mode de réalisation préféré, le procédé selon l'invention comprend en outre le mélange :

(iii) d'un second type d'enzyme capable d'effectuer des liaisons covalentes entre les monomères.

La concentration en monomères capables de former des polymères, optionnellement par des liaisons de faible énergie, la concentration en enzyme capable de dégrader lesdits polymères et la concentration en enzyme capable d'effectuer des liaisons covalentes entre lesdits monomères, pour obtenir un biomatériau qui présente le temps de gel et la durée de vie de gel souhaités, sont déterminées selon le protocole décrit dans les exemples.

Le procédé est réalisé à une température à laquelle les deux types d'enzymes sont actives. Avantageusement, le procédé est réalisé à une température comprise entre 0 et 100°C, de préférence entre 5 et 75°C, par exemple entre 10 et 50°C ou entre 15 et 45°C, et de manière particulièrement préférée entre 20 et 40°C.

Avantageusement, la quantité de monomères capables de former des polymères est comprise entre 0,1 et 30% en poids par rapport au poids total du biomatériau, de préférence entre 0,2 et 20%, préférentiellement encore entre 0,5 et 15%, et de manière particulièrement préférée entre 1 et 10%.

Avantageusement encore, le rapport de la concentration en enzyme capable d'effectuer des liaisons covalentes entre les monomères sur la concentration d'enzymes capables de dégrader les polymères est supérieur ou égal à 1, de préférence supérieur ou égal à 10, et de manière particulièrement préférée supérieur ou égal à 20.

De préférence, la concentration en enzyme capable d'effectuer des liaisons covalentes entre les monomères est supérieure à 0,001 U/ml, de préférence supérieure à 0,01 U/ml et de manière particulièrement préférée supérieure à 0,1 U/ml.

Selon le procédé de l'invention, la concentration en monomères capables de former des polymères, la concentration en enzyme capable de dégrader lesdits polymères et, éventuellement, la concentration en enzyme capable d'effectuer des liaisons covalentes entre lesdits monomères sont choisies de sorte que la résolution des équations (I), (II), (III) et (IV) suivantes :

$$\frac{dg}{dt} = -\frac{V_P}{K_P + g} \times g + \frac{V_T}{K_T + s} \times s + V_H \quad \textbf{(I)}$$

$$\frac{ds}{dt} = \frac{V_P}{K_P + g} \times g - \frac{V_T}{K_T + s} \times s - \frac{V'_P}{K_P + s} \times s - V_H \quad \textbf{(II)}$$

$$\frac{df}{dt} = \frac{V'_P}{K_P + s} \times s \quad \textbf{(III)}$$

$$g_t + s_t + f_t = S_0 \quad \textbf{(IV)}$$

lesquelles équations décrivent respectivement l'évolution du nombre de monomères liés (dg) (I), l'évolution du nombre

de monomères en solution (ds) (II) et l'évolution du nombre de monomères dégradés (df) en fonction du temps (dt) (III), et l'équation de conservation de masse (IV),
où :

- g correspond à la quantité de monomères sous forme liée,
- t correspond au temps,
- $V_P$ correspond à la vitesse de l'enzyme capable de dégrader les polymères exprimée en quantité de monomères liés ramenés sous leur forme libre par ladite enzyme et par unité de temps,
- $K_P$ représente la constante de Michaelis de l'enzyme capable de dégrader les polymères,
- s représente la quantité de monomères sous forme libre,
- $V_T$ correspond à la vitesse de l'enzyme capable d'effectuer des liaisons covalentes entre les monomères exprimée en quantité de monomères sous forme libre liés par ladite enzyme et par unité de temps,
- $K_T$ représente la constante de Michaelis de l'enzyme capable d'effectuer des liaisons covalentes entre les monomères,
- $V_H$ représente la vitesse de liaison, par des liaisons de faible énergie et par unité de temps, de monomères sous forme libre, dans le cas de monomères capables de polymériser par de telles liaisons,
- $V'_P$ correspond à la vitesse de l'enzyme capable de dégrader les monomères sous forme libre exprimée en quantité de monomères dégradés, lesquels ne peuvent plus polymériser, générés par ladite enzyme et par unité de temps,
- $g_t$ correspond à la quantité de monomères sous forme liée au temps t,
- $s_t$ correspond à la quantité de monomères sous forme libre au temps t,
- $f_t$ correspond à la quantité de monomères dégradés qui ne sont plus capables de former des polymères au temps t,
- $S_0$ correspond à la quantité initiale de monomères,

permet d'obtenir un temps de gel et la durée de gel souhaités.

Les inventeurs ont pu démontrer avec différents types d'enzymes, qu'il était possible d'obtenir des biomatériaux présentant une cinétique de transition solution/gel puis gel/solution déterminée.

Les constantes pour ces différentes enzymes sont bien connues de l'homme du métier. À titre d'exemple, on peut citer la base de données http://www.brenda.uni-koeln.de/. qui décrit de telles constantes enzymatiques. Dans tous les cas, l'homme du métier pourra déterminer simplement ces différentes constantes pour des enzymes données selon des méthodes bien connues, telles que celles décrites dans http://www.brenda.uni-koeln.de/ et dans PRICE and STEVEN, Fundamentals of Enzymology: The Cell and Molecular Biology of Catalytic Proteins, Oxford University Press.

Dans ces équations, VP et VT sont ainsi calculées selon les formules suivantes :

$V_P = k_{CAT-P}$ [P] et $V_T = k_{CAT-T}$ [T], où [P] et [T] représentent respectivement les concentrations en enzyme capable de dégrader les polymères et en enzyme capable d'effectuer des liaisons covalentes entre les monomères, et $k_{CAT-P}$ et $k_{CAT-T}$ représentent les constantes catalytiques pour ces enzymes.

Dans tous les cas, l'homme du métier pourra déterminer simplement et sans expérimentation excessive en suivant les protocoles décrits dans les exemples les différentes concentrations de monomères et d'enzyme pour obtenir un biomatériau présentant les cinétiques de transition successives solution/gel et gel/solution recherchées.

À titre d'exemple, un biomatériau comprenant 5% de gélatine de type A, à titre de monomère capable de former des polymères par des liaisons de faible énergie, de la thermolysine isolée à partir de *Bacillus thermoproteolyticus rokko,* de la transglutaminase isolée à partir de *Streptoverticillium sp* et présentant un temps de gel de 19 minutes et une durée de vie du gel de 743 minutes peut être obtenu à 40°C en utilisant 1 U/ml de transglutaminase et 0,0125 U/ml de thermolysine.

À titre d'exemple encore, un biomatériau comprenant 5% de gélatine de type A, à titre de monomère capables de former des polymères par des liaisons de faible énergie, de la thermolysine isolée à partir de *Bacillus thermoproteolyticus rokko,* de la transglutaminase isolée à partir de *Streptoverticillium sp* et présentant un temps de gel de 2 minutes et une durée de vie du gel supérieure à 5000 minutes peut être obtenu à 27°C en utilisant 1 U/ml de transglutaminase et 0,0125 U/ml de thermolysine.

Avantageusement, le procédé selon l'invention comprend en outre une étape de lyophilisation du biomatériau sous forme de gel.

D'autres caractéristiques de l'invention apparaîtront dans les exemples qui suivent, sans pour autant que ceux-ci ne constituent une quelconque limitation de l'invention.

**Exemple 1 Gel chimique :**

**1-1 développement d'un modèle théorique :**

Une étude de l'équilibre dynamique correspondant à la matrice extracellulaire a permis d'établir un modèle mathématique simplifié d'un tel système dynamique dans lequel on met en oeuvre deux réactions enzymatiques antagonistes. L'une est catalysée par une enzyme capable d'effectuer des liaisons covalentes entre les monomères solubles (s) pour obtenir un réseau de monomères liés (g). L'autre est catalysé par une enzyme (P) qui hydrolyse le réseau de monomères liés (g) en monomères solubles (s). Enfin, et dans certains cas, une troisième réaction aussi catalysée par l'enzyme (P) consiste en l'hydrolyse des monomères solubles en monomères dégradés (f) de taille trop faible pour participer au réseau ou qui ne peuvent plus servir de substrat à l'enzyme liant les monomères solubles (s) par des liaisons covalentes (T). Cette dernière réaction qui se traduit par une fuite de monomères du cycle est également ajoutée au modèle. Le modèle peut être simplement représenté selon le schéma réactionnel suivant :

Schéma I :

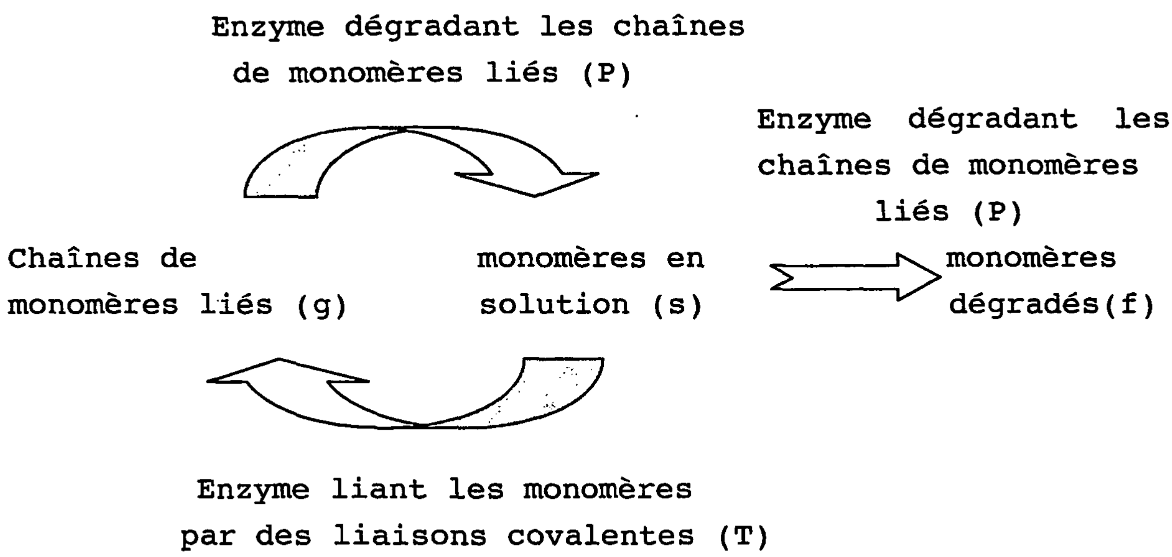


Ce mécanisme réactionnel peut être simplement décrit par les trois équations différentielles qui suivent, où les réactions enzymatiques sont assimilées à des réactions michaéliennes simples et par une quatrième équation de conservation de la masse.

L'équation différentielle permettant de décrire l'évolution du nombre de chaînes de monomères liés (g) en fonction du temps est la suivante:

$$\frac{dg}{dt} = -\frac{V_P}{K_P + g} \times g + \frac{V_T}{K_T + s} \times s$$

L'équation différentielle permettant de décrire l'évolution du nombre de monomères en solution (s) en fonction du temps est la suivante:

$$\frac{ds}{dt} = \frac{V_P}{K_P + g} \times g - \frac{V_T}{K_T + s} \times s - \frac{V'_P}{K_P + s} \times s$$

L'équation différentielle permettant de décrire l'évolution du nombre de monomères dégradés (f) en fonction du temps est la suivante:

$$\frac{df}{dt} = \frac{V'_P}{K_P + s} \times s$$

Enfin, l'équation de conservation de masse est la suivante:

$$g_t + s_t + f_t = S_0$$

Dans ces équations différentielles, $V_P$ et $V_T$ représentent les vitesses maximales pour les enzymes P et T respectivement, $K_P$ et $K_T$ représentent les constantes de Michaelis pour les enzymes P et T respectivement.

Dans ces équations, $V_P$ et $V_T$ sont calculées selon les équations suivantes :

$$V_P = k_{CAT-P}\,[P] \text{ et } V_T = k_{CAT-T}\,[T]$$

Dans la transposition de cette équation à un système *in vitro,* les différentes valeurs de [P], [T], $V_P$, $V_T$ ,$K_P$ et $K_T$ correspondent à des constantes.

Les concentrations en substrats sont calculées pour chaque enzyme à l'origine, elles sont ensuite déterminées au cours de la réaction à l'aide des différentes équations différentielles.

**1-2 : modélisation de la formation d'un gel de gélatine en présence d'une transglutaminase et de thermolysine :**

Ce modèle théorique a été appliqué à la formation d'un gel physique de protéine, et plus précisément de gélatine à 40°C en présence d'une transglutaminase bactérienne, qui lie les monomères de gélatine par des liaisons covalentes, et d'une protéase : la thermolysine.

La concentration de gélatine utilisée est de 5%, soit 50 $g.l^{-1}$. A partir de la séquence peptidique de la gélatine, il est possible de déterminer simplement la concentration en chaînes latérales, à savoir 0,45 $mol.l^{-1}$.

Enzymes utilisées :

Les différentes constantes pour la protéase et la transglutaminase sont connues dans la littérature ou peuvent être déterminées simplement selon des techniques bien connues de l'homme du métier (SEGEL, Wiley Classics Library, Enzyme Kinetics, 1993).

1-Protéase :

1.1- Source : La protéase utilisée est une métalloprotéase bactérienne, la thermolysine, commercialisée par SIGMA (REF : P-1512). Cette enzyme est une protéase de type X issue de *Bacillus thermoproteolyticus rokko* qui est une souche bactérienne thermophile. Cette enzyme reconnaît spécifiquement les résidus Ile, Leu, Val, Phe, Met et Ala. Très stable à température ambiante, cette enzyme présente une masse molaire de 34 kDa pour 316 acides aminés.

2.2- Activité : Pour la thermolysine, sa constante de Michaelis (de dissociation) est connue pour différents substrats protéiques et peptidiques dans la littérature (MATSUBARA et al., Biochem. Biophys. Res. Commun., vol. 21, p : 242-247, 1965). A partir de ces données, il est possible de déduire une valeur moyenne de $K_P$ égale à 1 mM. La littérature décrit également les valeurs de $k_{cat}$ de la thermolysine pour différents substrats (LIGNE et al., Biochim. Biophys. Acta., vol.1337, p :143-148, 1997). A partir de ces données, il est également possible de déterminer une valeur moyenne de $k_{cat}$ égale à 1000 $min^{-1}$. La spécificité de la thermolysine étant connue, l'analyse de la séquence de la gélatine permet de déterminer que, dans le système étudié, la concentration initiale en substrat pour la thermolysine est donc de 0,15 x 0,45 = 0,68 $mol.l^{-1}$ = 68 mM soit 68 $K_P$. Cette concentration est proche d'une

concentration saturante en substrat, le substrat n'est donc pas limitant dans la réaction de la thermolysine.

Les différentes constantes enzymatiques de cette enzyme ont également pu être mesurées en utilisant le N-(3-[2-furyl] acryloyl)-Glycine-Leucineamide(SIGMA) comme substrat, pour la formation de Furyl-acroyl-glycine et de Leucine-amide. La disparition du substrat au cours de l'hydrolyse se traduit par une diminution de la densité optique mesurée à 345 nm.

2-Transglutaminase :

2.1- Source : La transglutaminase utilisée est produite par la société AJINOMOTO sous la dénomination ACTIVA WM®. Cette enzyme bactérienne est produite dans le milieu de culture de *Streptoverticilium sp.* Cette enzyme correspond à une chaîne polypeptidique de 331 acides aminés pour un PM de 38,000 Da. Le résidu cystéine en position 64 correspond au site actif.

2.2- Activité : Cette enzyme reconnaît les résidus lysine et catalyse la formation de liaisons covalentes entre les chaînes latérales des résidus lysines et les chaînes latérales d'autres résidus. L'activité de cette enzyme est optimale à une température de 50-55°C (100 $U.g^{-1}$ à 40°C), et dans une large gamme de pH (activité de 4,5 à 9 avec un optimum entre 6 et 7).

L'activité de cette enzyme est mesurée d'après la quantité d'enzyme nécessaire pour catalyser la formation d'une micromole d'acide hydroxyamique pendant une minute à 40°C, à partir du Nα-CBZ-Gln-Gly (SIGMA) et de l'hydroxylamine. Le protocole permettant de déterminer l'activité de cette enzyme est le suivant :

| | |
|---|---|
| μl d'enzyme dans du tampon acétate à pH 6 | 400 μl |
| NH2OH 2M | 25 μl |
| Nα-CBZ 0,1M | 75 μl |
| 10 min à 37°C | |
| TCA 10% FeCL3 3% | 500 μl |
| Centrifugation 400g 15 min | |
| DO 525 nm (blanc avec solution sans enzyme) | |

L'activité est ensuite calculée par l'équation suivante :

Activité (U/mL/min)= [100 x (DO 525/ 238)]/Vol enzyme (ml)

Différentes réactions sur ce substrat ont permis de déterminer une constante de dissociation $K_T$ de 8 mM et un $k_{cat}$ de 100 $min^{-1}$ en utilisant la gélatine comme substrat. La gélatine contient seulement 4% de lysine, la concentration initiale en substrat pour la transglutaminase est donc de 17 mM.

Modélisation :

À partir des différentes valeurs déterminées précédemment, il est possible de suivre le comportement de la fraction liée en fonction du temps en appliquant la méthode d'Euler d'approximation des équations différentielles (BRONSON, Equations Différentielles, Mc Graw-Hill ed, 1994). Dans cette première modélisation, le rapport des activités entre la transglutaminase et la protéase est de 10 (avec une concentration en transglutaminase de 1 U/ml). On pose en outre $g_0$=0 et $s_0$=68 mM. Les données de la littérature montre qu'un gel de gélatine est obtenu par action de la transglutaminase lorsque 18% des liaisons théoriques sont effectivement réalisées (FUCHSBAUER et al., Biomaterials, vol.17, p : 1481-1488, 1999).

Les différentes modélisations effectuées montrent que la variation de $V'_P$ par rapport à $V_P$ est de faible importance et que la cinétique d'apparition des protéines liées montre une forte augmentation initiale jusque des valeurs proches de 100%, puis une décroissance très lente qui dépend de la vitesse d'apparition des petits fragments. Un exemple de modélisation de l'évolution de la fraction liée en fonction du temps, avec un rapport $V'_P / V_P$ de 0,1, est présenté dans la figure 1.

**1-3 : formation d'un gel de gélatine en présence d'une transglutaminase seule :**

Pour valider ce modèle théorique, les inventeurs ont d'abord effectué une première série d'expériences dans laquelle la formation d'un gel de gélatine a été suivie en présence de différentes quantités de transglutaminase uniquement.

Protéines polymérisables utilisées :

La gélatine de type A utilisée (G2500) est extraite à partir de peau de porc selon un protocole acide (SIGMA). Les concentrations de gélatine dans la solution sont exprimées en pourcentage (poids/volume). Ainsi, une solution de 1% de gélatine correspond à une solution de 1g de gélatine dans 100ml de solution. Les concentrations de gélatine utilisées ont varié dans les expériences entre 2 et 10% et la température de gélification était de 40°C.

La transglutaminase utilisée correspond à la transglutaminase décrite précédemment (voir 1-2). Les concentrations de transglutaminase utilisées dans ces expériences ont varié entre 0,1 U/ml et 1 U/mL.

Suivi des transitions solution/gel et gel/solution :

La formation d'un gel est suivie à l'aide d'un rhéomètre AR1000 (TA INSTRUMENT) ou RS 150 (THERMORHEO). Ces rhéomètres ont été montés avec des géométries de type cônes/plan de 60 mm et de 2° d'angle. Un cône en acier est utilisé sur l'AR1000 et un cône en titane est utilisé sur le RS150.

Ces rhéomètres permettent d'effectuer une analyse oscillatoire, ou analyse dynamique, qui consiste à imposer à l'échantillon un cisaillement oscillatoire de pulsation donnée ω. Au cours de ce mouvement, la contrainte et le gradient de cisaillement évoluent sinusoïdalement au cours du temps, avec la même pulsation, mais avec un certain déphasage de l'une par rapport à l'autre. Les rhéomètres permettent de mesurer de nombreux paramètres permettant de suivre la gélification. Le module de cisaillement ($G^*_{(\omega)}$) est un nombre complexe qui exprime la composante visqueuse et élastique de l'échantillon et répondant à la formule suivante :

$$G^*_{(\omega)} = G'_{(\omega)} + i\,G''_{(\omega)}$$

G'est appelé module de conservation (Storage Modulus) et G" est appelé module de perte (Loss Modulus). Ces deux modules sont exprimés en Pascals (Pa). Dans un liquide newtonien, le module de conservation est nul, seul le module de perte existe, c'est pourquoi le module de perte est parfois également qualifié de module visqueux. En revanche, seul le module de conservation existe dans un solide élastique, c'est pourquoi il est parfois qualifié de module élastique. On peut relier le module de cisaillement avec les modules de perte et de conservation selon les formules suivantes :

$$G' = G^* \cos \delta \qquad \text{et } G'' = G^* \sin \delta$$

Dans lesquelles δ représente le déphasage entre la contrainte et la déformation de cisaillement. A partir de ces formules, on peut déduire la relation suivante :

$$\mathrm{Tan}\ \delta = G'' / G'$$

Tan δ devient alors une valeur pertinente permettant de caractériser la gélification de l'échantillon. Lorsque l'échantillon est liquide G" > G' et Tan δ >1. A l'inverse, l'échantillon est gélifié lorsque G" < G' et Tan δ <1. Le temps mis pour atteindre la valeur Tan δ =1 représente « le temps de gel ». Dans les différentes expériences qui suivent, le rapport des modules G" / G' pour différentes solutions est exprimé en fonction du temps, avec la valeur de G" / G' = 1

correspondant au « point de gel ».

Résultats :

L'évolution des propriétés viscoélastiques, suivies à l'aide d'un rhéomètre, d'une solution de gélatine à 5% à 40°C et en présence de différentes concentrations de transglutaminase (0,1, 0,15, 0,2, 0,5 et 1 U/ml) est présentée dans la figure 2. On observe que, dans toutes les conditions testées, l'échantillon est bien liquide au début de l'expérience (G"/G' < 1), puis qu'un gel se forme (G"/G' > 1) et évolue au cours du temps. Les expériences montrent en outre qu'après le point de gel et pour n'importe quelle concentration d'enzyme, le module G" atteint un plateau autour de 1 Pa. Les valeurs du module G' sont donc dépendantes de la concentration en enzyme. Les variations du rapport G"/G' observées après le point de gel reflètent donc les variations de G'.

La figure 3 représente la vitesse de gélification (inverse du temps de gel) des solutions de gélatine à 5% à 40°C en fonction de la concentration en transglutaminase. Les résultats montrent que la courbe de gélification est conforme au modèle théorique, incluant uniquement l'étape avec la transglutaminase, et ceci jusqu'au point de gel où G" / G' = 1.

La figure 4 représente la vitesse de réaction enzymatique en fonction de la concentration d'enzyme à 40°C, après le point de gel. Les résultats montrent qu'au-delà du point de gel la courbe de gélification n'est plus conforme au modèle théorique. On observe alors que les courbes ne sont plus extrapolables par régression linéaire, mais suivent un comportement en loi de puissance d'équation $y= 15x^{1,85}$ (courbe en pointillés sur la figure) avec un $R^2$ de 0,98. Après le point de gel, la réaction enzymatique est dans un régime limité par la diffusion de l'enzyme dans le gel. L'impact de la diffusion se matérialise dans cette équation de vitesse par un coefficient de 1,85 qui affecte la concentration de transglutaminase.

La figure 5 montre l'évolution, en fonction du temps, de la quantité de transglutaminase active dans le gel en tenant compte de la diffusion pour 1 unité (■) et 0,4 unité (♦) d'enzyme en solution. Ces expériences ont donc permis de montrer qu'après la formation du gel, la valeur du coefficient de diffusion pour la transglutaminase évolue progressivement jusqu'à atteindre la valeur de 1,85. Cette évolution du coefficient de diffusion peut être déterminée simplement à partir de cette courbe et en fonction du temps.

Il semble donc que l'action enzymatique lors de la gélification suive deux régimes successifs. Le premier, de l'état liquide jusqu'aux alentours du point de gel, n'est pas limité par la diffusion et les règles d'enzymologie classique peuvent s'appliquer. La vitesse de réaction est alors une fonction linéaire de la concentration en enzyme. Le second, après le point de gel, est un milieu non conventionnel où la vitesse de diffusion de l'enzyme n'est plus négligeable et limite la vitesse de réaction apparente.

Des expériences effectuées sur des gels de gélatine de 2 et de 10% en présence de différentes concentrations de transglutaminase ont permis de confirmer un comportement similaire au cours de la gélification. Dans ces conditions, chaque type de gel bénéficie d'un coefficient de diffusion spécifique qui peut être déterminé simplement comme précédemment.

**1-4 nouvelle modélisation de la formation d'un gel de gélatine en présence d'une transglutaminase et de thermolysine:**

Un gel de gélatine a été modélisé en utilisant les mêmes paramètres que ceux décrits précédemment (voir 1-2) et en utilisant rapport $V_T/V_P$ de 10 et un rapport $V'_P/V_P$ de 0,05. Toutefois, l'effet de contrainte diffusionnelle d'un gel de gélatine à 5% sur la vitesse de réaction de l'enzyme a été intégré dans les équations différentielles décrites précédemment (voir 1-2). Ainsi, la concentration en transglutaminase a été affectée d'un exposant de 1,85 dans les équations rendant compte de l'équilibre réactionnel dès lors que le point de gel est dépassé selon la formule suivante :

$$V_T = k_{CAT-T}\ [T_0]^{1,85}$$

Des études réalisées précédemment ont permis de montrer que la diffusion contrôlait l'hydrolyse des gels uniquement en présence de très faible concentration d'enzyme, à savoir inférieur à 1 nM (FADDA et al., Biophys. J., vol.85(5), p :2808-2817, 2003), mais pas à des concentrations plus importantes (BERRY et al., Biochim. Biophys. Acta., vol.1524, p :110-117, 2000 ; GIRAUDIER et al., Biomacromolecules, vol.5(5), p :1662-1666, 2004). En conséquence, aucun coefficient n'a été appliqué à la concentration de thermolysine dans les équations.

La figure 6 représente le résultat d'une modélisation de l'évolution de la fraction liée en fonction du temps avec une limitation diffusionnelle et un rapport de $V_T/V_P$ de 10 (concentration en transglutaminase de 1U/ml). On observe que, d'après cette modélisation, le milieu protéique effectue successivement une transition solution/gel, avec un premier

passage du point de gel à 80 minutes, puis une transition gel/solution, avec un deuxième passage du point de gel à 330 minutes. Selon le modèle établi par les inventeurs, le milieu protéique susceptible d'être obtenu présente des propriétés nouvelles, avec une capacité à effectuer successivement une transition solution/gel puis gel/solution, avec un état de gel maintenu pendant une durée de 250 minutes.

Pour autant, l'effet diffusionnel a été considéré comme tout ou rien, à savoir sans limitation avant la transition de gélification et avec une limitation totale au delà de cette transition. Les expériences réalisées précédemment (voir 1-3) ont permis de montrer que les contraintes diffusionnelles augmentent au fur et à mesure que le réseau de lien covalent se développe dans le gel. Cet effet progressif a été pris en compte dans le modèle en utilisant la formule suivante :

$V_T = k_{CAT\text{-}T}\,[T_0]^{\alpha}$ où $\alpha$ varie en fonction du temps de façon hyperbolique de 1,1 à 1,85 (voir figure 5).

La figure 7 représente le résultat d'une modélisation de l'évolution de la fraction liée en fonction du temps avec une limitation diffusionnelle progressive et différents rapports $V_T/V_P$ (concentration en transglutaminase de 1U/ml). Les résultats montrent là encore des transitions solution/gel, puis gel/solution pour les différents rapports $V_T/V_P$ représentés. Pour le rapport $V_T/V_P$ de 10, le milieu protéique atteint le point de gel en 15 minutes, le gel effectue ensuite une transition gel/solution après 165 minutes. Par rapport à la modélisation précédente, la prise en compte d'une limitation diffusionnelle progressive prédit l'obtention d'un gel pendant 150 minutes, au lieu de 250 minutes précédemment.

**<u>1-5 : formation d'un gel de gélatine en présence de transglutaminase et de thermolysine simultanément :</u>**

Pour valider le modèle théorique décrit précédemment (voir 1-4), différentes expériences ont été effectuées en utilisant de la gélatine et de la transglutaminase dans les mêmes conditions que celles utilisées précédemment (voir 1-3). En outre, une protéase, la thermolysine a également été associée à la solution de départ. La formation de gels a été suivie en fonction du temps et à 40°C en fonction des concentrations de transglutaminase et de thermolysine utilisées. Les résultats obtenus, en comparaison des prédictions du modèle théorique, sont représentés dans le tableau I suivant :

**Tableau I**

| Rapport Tgase / Thermo-lysine | Tgase (en U/ml) | Thermolysine (en U/ml) | théorique | | | Expérimental | | |
|---|---|---|---|---|---|---|---|---|
| | | | Formation d'un gel | Temps de gel (min) | Durée de vie du gel (min) | Formation d'un gel | Temps de gel (min) | Durée de vie du gel (min) |
| 10 | 0,4 | 0,04 | oui | 35 | 270 | non | - | - |
| 10 | 1 | 0,1 | oui | 15 | 150 | non | - | - |
| 60 | 0,4 | 0,0067 | oui | 35 | 1600 | non | - | - |
| 60 | 1 | 0,0167 | oui | 15 | 875 | non | - | - |
| 75 | 0,4 | 0,0053 | oui | 35 | 2000 | non | - | - |
| 75 | 1 | 0,013 | oui | 15 | 1150 | non | - | - |
| 80 | 0,4 | 0,005 | oui | 40 | 3000 | oui | 56 | > 4800 |
| 80 | 1 | 0,0125 | oui | 15 | 1200 | oui | 19 | 743 |

Les résultats montrent que, conformément au modèle théorique développé, il est possible d'obtenir expérimentalement à partir d'une solution de monomères polymérisables par des liaisons covalentes en présence de deux activités enzymatiques antagonistes (dans ce cas une solution de gélatine à 5% en présence de différentes concentrations de transglutaminase et de thermolysine) une transition solution/gel puis une transition gel/solution successivement.

Si un tel gel est obtenu dans toutes les simulations au bout d'un temps qui ne dépend que de l'activité Transglutaminase, les différentes expériences montrent qu'il existe un rapport $V_T/V_P$ qui constitue une valeur seuil pour la première transition solution gel. Dans le cas d'une solution de gélatine à 5% en présence de transglutaminase et de thermolysine, le rapport $V_T/V_P$ critique pour obtenir cette première transition solution/gel est compris entre 75 et 80.

Les différentes expériences confirment en outre le temps de gel et la durée de vie du gel dépendent de la concentration en transglutaminase et en thermolysine respectivement.

La figure 7 montre l'évolution expérimentale des propriétés viscoélastiques de solutions de gélatine à 5% en présence de différentes concentrations de transglutaminase et de thermolysine à 40°C.

Au regard du modèle théorique développé, il est donc possible d'obtenir un biomatériau capable d'effectuer

successivement une transition solution/gel puis gel/solution. Pour cela, il suffit de déterminer expérimentalement le rapport critique enzyme liant les monomères/ enzyme dégradant les monomères liés pour obtenir cette première transition solution/gel en fonction du monomère et des enzymes utilisés. Le modèle théorique permet ensuite d'adapter au moins les différentes concentrations enzymatiques de sorte d'obtenir un gel qui présente d'une part le temps de gel et d'autre part la durée de vie souhaités.

Des expériences complémentaires ont été réalisées, dans lesquelles la gélatine 5% est additionnée de 1% d'alginate ou d'oligoalginate. On obtient là encore une transition solution/gel puis gel/solution.

**1-6 formation d'un gel de gélatine en présence, simultanément, de transglutaminase et de différentes protéases :**

Pour confirmer le modèle développé, différentes protéases ont été utilisées.

La trypsine, EC 3-4-21-4, est une sérine protéase qui hydrolyse les liaisons peptidiques en aval des lysines et arginines. La trypsine utilisée est d'origine pancréatique de bovin et commercialisée par Sigma (T-1426). L'activité de cette enzyme est mesurée d'après la quantité d'enzyme nécessaire pour catalyser l'hydrolyse du p-Tosyl-Arginie-méthyl-Ester (Sigma T4626).

La collagénase (EC 3.4.24.3) hydrolyse spécifiquement les liaisons peptidiques X-Gly des séquences Pro-X-Gly-Pro, que l'on retrouve notamment dans les chaînes $\alpha$ du collagène. La collagénase utilisée est obtenue à partir de Clostridium histolyticum Type IA et commercialisée par Sigma (C-9891). L'activité de cette enzyme est mesurée d'après la quantité d'enzyme nécessaire pour catalyser l'hydrolyse du N-(3-[2-furyl]acryloyl)-Leu-Gly-Pro-Ala ou FALGPA (Sigma F-5135).

Ces activités enzymatiques étant mesurées sur l'hydrolyse de peptides de synthèse portant un groupement chromophore, pour une meilleure comparaison, l'activité de la thermolysine a été mesurée sur l'hydrolyse de N-(3-[2-furyl]acryloyl)-Gly-Leu-amide (Sigma N-7383). C'est cette activité qui est reportée dans les tableaux avec plusieurs protéases.

Pour les gels, la concentration de gélatine était de 7% et la température de gélification était de 40°C. La transgglutaminase utilisée correspond à la transglutaminase décrite précédemment. Le suivi des gels avec le temps a été effectué comme en 1-5. Les résultats obtenus sont représentés dans le tableau II suivant :

Tableau II

| Rapport Tgase / Protéase | Tgase (en U/ml) | Protéase (en U/ml) | Expérimental | | |
|---|---|---|---|---|---|
| | | | Formation d'un gel | Temps de gel (min) | Durée de vie du gel (min) |
| 375 | 1,5 | 0,004(16 nM thermolysine) | oui | 24 | 546 |
| 48 | 1,5 | 0,031(31 nM trypsine) | oui | 14 | 396 |
| 520 | 1,5 | 0,003(16 nM collagénase) | oui | 8 | 492 |

Les résultats montrent donc que le modèle est généralisable à d'autres protéases.

**Exemple 2 : Gel physique :**

**2-1 : développement d'un modèle théorique :**

Ce modèle rend compte de l'équilibre dynamique observé dans le cas de la formation d'un gel physique uniquement et en présence d'une seule activité enzymatique. La gélification résulte alors uniquement de la formation de liaisons de faible énergie (Van der Waals, liaisons hydrogènes, hydrophobes, etc. ; H) entre des monomères en solution (s). Dans le cas de protéines par exemple, leurs propriétés de polyélectrolytes font qu'elles sont quasiment toutes capables de former des gels physiques. La dégradation des polymères correspondant aux chaînes de monomères liés (g) du gel est catalysée par une enzyme (P) qui les hydrolyse en monomères solubles (s). Enfin, il existe dans certains cas une troisième réaction aussi catalysée par l'enzyme (P) qui consiste en l'hydrolyse des monomères solubles en monomères dégradés (f) de taille trop faible pour participer au réseau ou ne pouvant plus former de liaisons avec d'autres monomères solubles. Cette dernière réaction qui se traduit par une fuite de monomères du cycle est également

ajoutée au modèle. Le modèle peut être simplement représenté selon le schéma réactionnel suivant :

Schéma II :

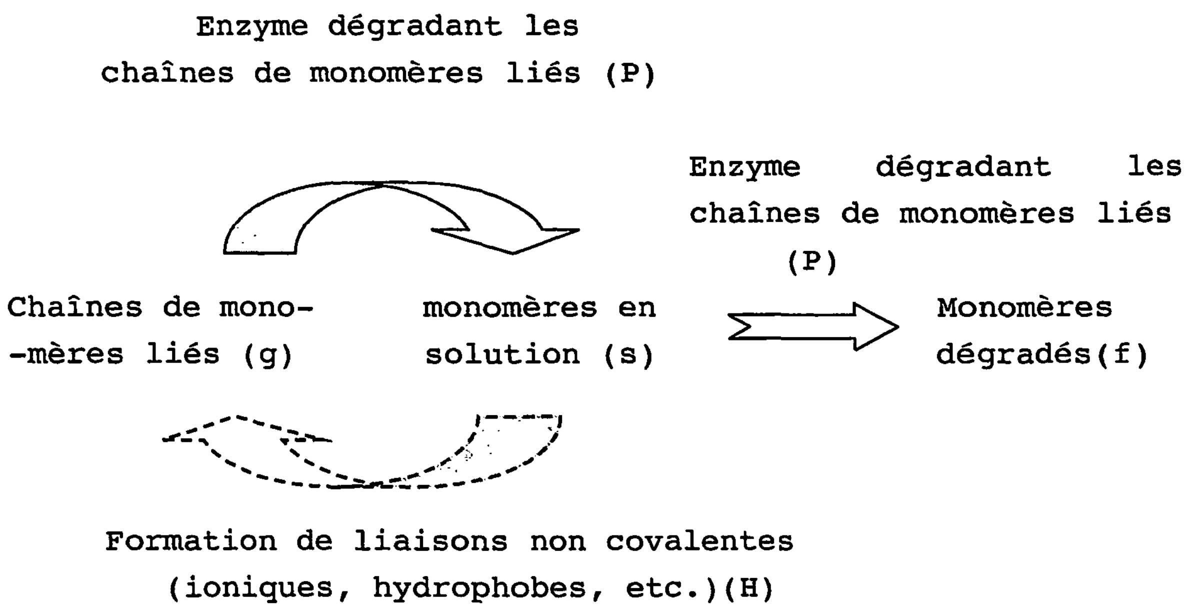


Dans ce deuxième cas, le mécanisme réactionnel peut être simplement décrit par les trois équations différentielles qui suivent, où les réactions enzymatiques sont assimilées à des réactions michaéliennes simples et par une quatrième équation de conservation de la masse.

L'équation différentielle permettant de décrire l'évolution du nombre de chaînes de monomères liés (g) en fonction du temps est la suivante:

$$\frac{dg}{dt} = -\frac{V_P}{K_P + g} \times g + V_H$$

L'équation différentielle permettant de décrire l'évolution du nombre de monomères en solution (s) en fonction du temps est la suivante:

$$\frac{ds}{dt} = \frac{V_P}{K_P + g} \times g - \frac{V'_P}{K_P + s} \times s - V_H$$

L'équation différentielle permettant de décrire l'évolution du nombre de monomères dégradés (f) en fonction du temps est la suivante:

$$\frac{df}{dt} = \frac{V'_P}{K_P + s} \times s$$

Enfin, l'équation de conservation de masse reste inchangée:

$$g_t + s_t + f_t = S_0$$

Dans ces équations différentielles, $V_P$ représente la vitesse maximale pour l'enzyme dégradant les monomères et $K_P$ représente la constante de Michaelis pour cette même enzyme.

Dans ces équations différentielles, $V_P$ est calculée selon l'équation suivante :

$$V_P = k_{cat-P}[P_0]$$

En outre, $V_H$ qui représente la vitesse de formation des liaisons de faible énergie, dépend du monomère utilisé.

Dans la transposition de cette équation à un système *in vitro,* les différentes valeurs de [P], $V_P$, $V_H$ et $K_P$ correspondent à des constantes.

Les concentrations en substrats sont calculées à l'origine, elles sont ensuite déterminées au cours de la réaction à l'aide des différentes équations différentielles.

**2-2 modélisation de la formation d'un gel physique de gélatine à 27°C en présence de thermolysine :**

Ce modèle théorique a été appliqué à la formation d'un gel physique de protéines, et plus précisément de gélatine à 27°C, et en présence de thermolysine. A cette température, la gélatine forme un gel par association partielle des monomères en triples hélices. Ces triples hélices, stabilisées par des liaisons hydrogènes, apparaissent par abaissement de la température.

Gélatine :

1-Descriptif : La gélatine est obtenue à partir de collagène. Le collagène est synthétisé sous forme de précurseurs de grande taille : les prochaînes α. La formation de procollagène s'accompagne d'un assemblage de trois prochaînes α par des liaisons hydrogène et de l'hydroxylation de certains résidus proline et lysine pour former de l'hydroxyproline et de l'hydroxylysine respectivement. Enfin, après sécrétion du procollagène dans le milieu extracellulaire, le procollagène est maturé par clivage des propeptides pour former le collagène. Ce dernier est alors susceptible de s'associer avec d'autres molécules de collagène pour former des fibrilles de collagène, lesquelles s'aggrègent pour former des fibres de collagène. La stabilité de la triple hélice formée par l'association des trois prochaînes α est fonction de l'organisme dont elle est issue. La température de dénaturation (Tm) va ainsi de 41,5°C pour certains mammifères à 6°C pour certains poissons vivants dans les eaux de l'Arctique. La variation de Tm est corrélée au taux d'hydroxyproline dans la molécule (PERSIKOV et al., Biochemistry, vol.39, p :14960-14967, 2000).

La gélatine est obtenue par un traitement acide ou alcalin des tissus contenant du collagène, ce qui aboutit à la dénaturation de la triple hélice du collagène (PEZRON, Physical Networks., Londres Elsevier Applied Science, 1990). Dans cette étude, les gélatines utilisées correspondent à des gélatines de type A obtenues par un traitement acide.

Lorsque la solution de gélatine semi-diluée est refroidie en dessous de la température de dénaturation des chaînes de collagène, un gel se forme. Les chaînes s'associent alors pour former des portions de triples hélices semblables à celles du collagène natif. Cette transition conformationnelle permet la création d'un réseau tridimensionnel qui piège les molécules d'eau présentes. Le gel formé est alors un gel physique, les triples hélices ne sont stabilisées que par des liaisons hydrogènes (liaisons faibles). Ces liaisons sont déstabilisées en augmentant la température ce qui provoque la dissolution du gel : ce phénomène de gélification est donc réversible.

Des études précédentes ont montré que l'élasticité de ce gel est liée uniquement à la concentration en hélices de l'échantillon (JOLY-DUHAMEL et al., Langmuir, vol.18, p :7158-7166 ,2002 ; JOLY-DUHAMEL et al., Langmuir, vol. 18, p :7208-7217 ,2002).

2- Concentration : La concentration en gélatine utilisée est la même que celle déterminée à l'exemple 1.

3- Vitesse de formation des hélices : La détermination de la vitesse de formation des triples hélices ($V_H$) a été effectuée en suivant le changement de conformation pelote/hélice par polarimétrie en fonction du temps ou de la température. En effet, la légère torsion résultant de la formation de la triple hélice entraîne une légère rotation d'une lumière polarisée traversant l'échantillon. Cette étude a été effectuée à l'aide d'un polarimètre PERKIN ELMER 341 ou d'un polarimètre JASCO 1100. Les mesures ont été effectuées à 436 nm.

L'étude par polarimétrie de la cinétique de formation de gel de gélatine de type A (type A ou AP1) a permis de déterminer, pour chaque gélatine, l'évolution du pourcentage d'hélices en fonction du temps et à 27°C. La figure 9 montre l'évolution du pourcentage d'hélices de solutions à 5% des deux gélatines de type A utilisées (type A et AP1)

en fonction du temps et avec un refroidissement de 40°C à 27°C à une vitesse de 0,5°C/minutes.

Les résultats que les cinétiques d'apparition sont globalement identiques dans les deux échantillons. Au temps de gel, le pourcentage d'hélices est identique pour les deux gélatines (6,4% à 40 minutes).

La vitesse de formation des hélices peut être calculée simplement à partir de la figure 9. La figure 10 montre également l'évolution du pourcentage d'hélices en fonction du temps et de la température pour un gel physique sans transglutaminase (carrés pleins) et pour un gel chimique obtenu en présence de transglutaminase (cercles vides). La vitesse d'apparition des hélices a été déterminée sur différents intervalles de temps et utilisée dans le modèle. Ainsi, pour la figure 10 et pour le gel physique, $V_H$ est de 45 $10^{-5}$ $M.min^{-1}$ pendant 10 minutes, 21 $10^{-5}$ $M.min^{-1}$ pendant 10 minutes, 9 $10^{-5}$ $M.min^{-1}$ pendant 20 minutes, puis de 4,5 $10^{-5}$ $M.min^{-1}$ pendant les 90 minutes suivantes et enfin de 2,5 $10^{-5}$ $M.min^{-1}$.

Des expériences similaires ont été effectuées sur des gels de gélatine de 2 à 10%.

Enzymes :

La protéase utilisée correspond à la thermolysine utilisée à l'exemple 1. Les paramètres utilisés sont donc les mêmes que précédemment. Toutefois, il a été tenu compte de la sensibilité différente des deux enzymes à cette température :

- Pour TL ($V_{i40°C}$= 1,66$V_{i27°C}$),
- Pour TG ($V_{i40°C}$= 2,68$V_{i27°C}$).

Modélisation :

A partir des différentes valeurs déterminées précédemment, il est possible de suivre le comportement de la fraction liée en fonction du temps en appliquant la méthode d'approximation d'Euler pour résoudre les équations différentielles comme précédemment. Plusieurs expériences de modélisation ont été effectuées en utilisant différentes concentrations en thermolysine.

La figure 11 présente quelques exemples de modélisation de l'évolution du pourcentage de chaînes liées en présence de différentes concentrations de thermolysine à 27°C (le seuil de gel correspond à 6,4% d'hélice). Les résultats obtenus montrent que pour certaines valeurs de $V_P$ une transition est possible et que, dans tous les cas, la fuite due à l'hydrolyse de petits fragments mène à la dégradation du réseau et donc à la solubilisation du gel formé.

**2-3 : formation d'un gel physique de gélatine en présence de thermolysine :**

Pour valider ce modèle théorique, différentes expériences ont été effectuées selon le même protocole que celui décrit précédemment (voir 2-2) et en ajoutant différentes concentrations de thermolysine. Les résultats obtenus, en comparaison des prédictions du modèle théorique, sont représentés dans le tableau III suivant :

Tableau III

| Thermolysine (en U/ml) | théorique | | | Expérimental | | |
|---|---|---|---|---|---|---|
| | Formation d'un gel | Temps de gel (min) | Durée de vie du gel (unités) | Formation d'un gel | Temps de gel (min) | Durée de vie du gel (min) |
| 0,1 | non | | | | | |
| 0,02 | non | | | | | |
| 0,015 | non | | | | | |
| 0,0125 | oui | 110 | 5000 | | | |
| 0,01 | oui | 110 | 6000 | non | - | - |
| 0,004 | | 110 | 20 000 | oui | 45 | env. 4300 |
| 0,0085 | | 110 | 9000 | oui | 68 | 254 |

Les résultats montrent là encore que, conformément au modèle théorique développé, il est possible d'obtenir

expérimentalement une transition solution/gel puis une transition gel/solution successivement, et ceci à partir d'une solution de monomères capables de polymériser par la formation de liaisons de faible énergie et en présence d'une enzyme capable de dégrader les chaînes de polymères liés (dans ce cas, une solution de gélatine à 5% et à 27°C en présence de différentes concentrations de thermolysine).

On observe qu'à toutes les concentrations de thermolysine testées (de 0,001 à 0,1 U/ml), le gel, s'il s'est formé, finit par fondre, ce qui est en accord avec le modèle théorique développé.

Dans le cas d'une solution de gélatine à 5% à 27°C et en présence de thermolysine, la concentration en thermolysine critique pour obtenir une première transition solution/gel est de 0,01 unité.

Le temps de gel dépend alors également de la concentration en thermolysine utilisée.

La figure 12 montre l'évolution expérimentale des propriétés viscoélastiques de solution de gélatine à 5% en présence de différentes concentrations de thermolysine et à 27°C.

Au regard du modèle théorique développé, il est donc possible d'obtenir un biomatériau capable d'effectuer successivement une transition solution/gel puis gel/solution. Pour cela, il suffit de déterminer la concentration critique en enzyme capable de dégrader les chaînes de monomères liées par des interactions de faible énergie. Le modèle théorique permet ensuite d'adapter la concentration enzymatique pour obtenir un gel qui présente d'une part le temps de gel et d'autre part la durée de vie souhaités.

__Exemple 3 Gel mixte :__

__3-1. développement d'un modèle théorique :__

Ce modèle intègre correspond à l'équilibre dynamique obtenu par la formation d'un gel mixte (physique et chimique) en présence en présence de deux activités enzymatiques antagonistes. Le modèle peut être simplement représenté selon le schéma réactionnel suivant (intégrant les données des schémas I et II):

__Schéma III :__

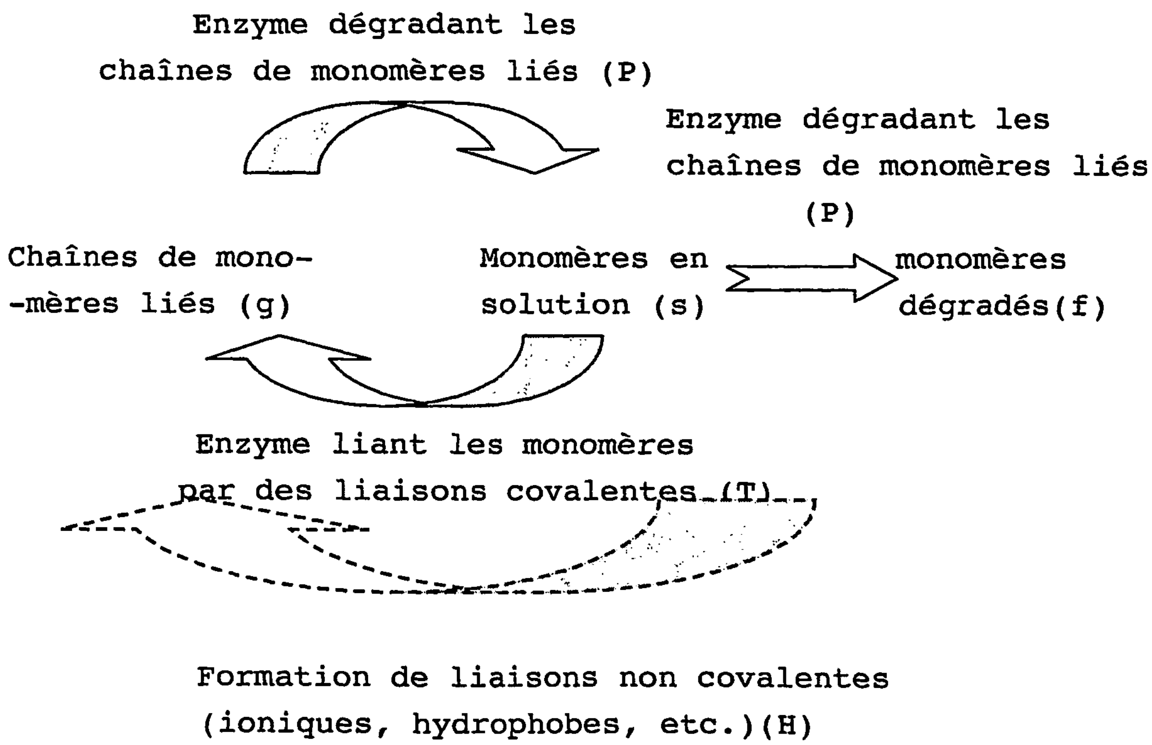


Dans ce troisième cas, le mécanisme réactionnel peut être simplement décrit par les trois équations différentielles qui suivent, où les réactions enzymatiques sont assimilées à des réactions michaéliennes simples et par une quatrième équation de conservation de la masse.

L'équation différentielle permettant de décrire l'évolution du nombre de chaînes de monomères liés (g) en

fonction du temps est la suivante:

$$\frac{dg}{dt} = - \frac{V_P}{K_P + g} \times g + \frac{V_T}{K_T + s} \times s + V_H$$

L'équation différentielle permettant de décrire l'évolution du nombre de monomères en solution (s) en fonction du temps est la suivante:

$$\frac{ds}{dt} = \frac{V_P}{K_P + g} \times g - \frac{V_T}{K_T + s} \times s - \frac{V'_P}{K_P + s} \times s - V_H$$

L'équation différentielle permettant de décrire l'évolution du nombre de monomères dégradés (f) en fonction du temps est la suivante:

$$\frac{df}{dt} = \frac{V'_P}{K_P + s} \times s$$

Enfin, l'équation de conservation de masse est la suivante:

$$g_t + s_t + f_t = S_0$$

Dans ces équations différentielles, $V_H$ représente la vitesse de formation des liaisons de faible énergie et dépend du monomère utilisé, $V_P$ et $V_T$ représentent les vitesses maximales pour l'enzyme P et T respectivement, $K_P$ et $K_T$ représentent les constantes de Michaelis pour l'enzyme P et T respectivement.

Dans la transposition de cette équation à un système *in vitro,* les différentes valeurs de [P], [T], $V_P$ ,$V_T$ ,$K_P$ et $K_T$ correspondent à des constantes.

**<u>3-2 modélisation de la formation d'un gel mixte de gélatine à 27°C en présence de transglutaminase et de thermolysine</u>** :

Ce modèle théorique a été appliqué à la formation d'un gel mixte de protéine, et plus spécifiquement de gélatine à 27°C et en présence de transglutaminase et de thermolysine.

<u>Gélatine :</u>

La concentration en gélatine utilisée est la même qu'aux exemples 1 et 2. La vitesse de formation des hélices est la même que celle déterminée à l'exemple 2.

<u>Enzymes :</u>

La protéase utilisée correspond à la thermolysine utilisée dans les exemples 1 et 2.

La transglutaminase est la même que celle utilisée dans l'exemple 1. Les coefficients de diffusion ont été intégrés comme décrit à l'exemple 1 pour tenir compte de l'effet de la diffusion sur l'activité de la transglutaminase.

Les paramètres utilisés pour ces deux enzymes sont donc les mêmes que ceux décrits précédemment.

<u>Modélisation :</u>

A partir des différentes valeurs déterminées précédemment, il est possible de suivre le comportement de la

fraction liée en fonction du temps en appliquant la méthode d'Euler d'approximation des équations différentielles comme précédemment. Plusieurs expériences de modélisation ont été effectuées en utilisant différentes concentrations en transglutaminase et en thermolysine. Un coefficient de diffusion a été appliqué pour la transglutaminase comme décrit à l'exemple 1.

La figure 13 montre l'évolution de la fraction de monomères liés en fonction du temps et pour différents rapports $V_T/V_P$ (avec une concentration en transglutaminase de 1 U/ml).

Les résultats suggèrent d'une part que la gélification est plus rapide que dans les exemples précédents, et d'autre part que la fraction de monomères liés et la stabilité des gels sont plus importantes que dans les gels physiques ou chimiques.

**3-3 : formation d'un gel mixte de gélatine en présence de thermolysine :**

Pour valider le modèle théorique décrit précédemment, une expérience a été effectuée sur un échantillon de gélatine à 5% et à 40°C, en présence des deux enzymes transglutaminase et thermolysine avec un rapport TransglutaminaseG/Thermolysine de 80 et une concentration de transglutaminase de 1 U/ml. La solution a été refroidie de 40°C à 27°C au rythme de 0,5°C par minute. L'évolution de la solution de gélatine est suivie simultanément en rhéologie et en polarimétrie comme décrit précédemment. Le résultat obtenu, en comparaison des prédictions du modèle théorique, est représenté dans le tableau IV suivant :

Tableau IV

| Rapport Tgase sur Thermolysine | Tgase (en U/ml) | Thermolysine (en U/ml) | théorique | | | Expérimental | | |
|---|---|---|---|---|---|---|---|---|
| | | | Formation d'un gel | Temps de gel (min) | Durée de vie du gel (min) | Formation d'un gel | Temps de gel (min) | Durée de vie du gel (min) |
| 80 | 1 | 0,0125 | oui | <5 | 15 000 | Oui | 2 | > 5000 |
| 80 | 0,4 | 0,005 | oui | 15 | > 17 000 | nd | | |
| 50 | 1 | 0,02 | oui | <5 | 5000 | nd | | |
| 50 | 0,4 | 0,008 | Oui | 15 | 9000 | nd | | |
| 10 | 1 | 0,1 | Oui | <5 | 750 | nd | | |
| 10 | 0,4 | 0,04 | oui | 15 | 1000 | nd | | |
| nd : non déterminé | | | | | | | | |

Les résultats montrent que, conformément au modèle théorique développé, il est possible d'obtenir expérimentalement à partir d'une solution de monomères polymérisables par des liaisons de faible énergie et par des liaisons covalentes, et en présence de deux activités enzymatiques antagonistes (dans ce cas, une solution de gélatine à 5%, à 27°C et en présence de différentes concentrations de transglutaminase et de thermolysine) une transition solution/gel puis une transition gel/solution successivement.

Conformément au modèle théorique développé, le temps de formation du gel est très rapide. En outre, la stabilité et la durée de vie du gel « mixte » obtenu est très importante au regard des gels physiques et également chimiques.

La figure 14 montre l'évolution simultanée des propriétés viscoélastiques et du pourcentage d'hélice d'une solution de gélatine à 5% en présence de transglutaminase et de thermolysine, avec un rapport Transglutaminase/thermolysine de 80 et une concentration en transglutaminase de 1U/ml, et à 27°C.

Au regard du modèle théorique développé, qui est basé sur les deux modèles théoriques précédents, il est possible d'obtenir un biomatériau capable d'effectuer successivement une transition solution/gel puis gel/solution. Pour cela, il suffit de déterminer expérimentalement le rapport critique enzyme liant les monomères/ enzyme dégradant les monomères liés comme dans l'exemple 1 pour obtenir une première transition solution/gel en fonction du monomères et des enzymes utilisés. Le modèle théorique permet ensuite d'adapter les différentes concentrations enzymatiques de sorte d'obtenir un gel qui présente d'une part le temps de gel et d'autre part la durée de vie souhaités.

**3-4 : formation d'un gel physique de gélatine en présence de différentes protéases** :

Pour confirmer les résultats obtenus au 3-3, des expériences ont été effectuées sur des échantillons de gélatine à 7% et à 40°C, en présence de transglutaminase et de différentes protéases avec une concentration de transglutaminase de 1,5 U/ml. La solution a été refroidie de 40°C à 27°C au rythme de 0,5°C par minute. L'évolution de la solution de gélatine est suivie simultanément en rhéologie et en polarimétrie comme décrit précédemment. Les résultats obtenus sont représentés dans le tableau V suivant :

Tableau IV

| Rapport Tgase sur Protéase | Tgase (en U/ml) | Protéase (en U/ml) | Expérimental | | |
|---|---|---|---|---|---|
| | | | Formation d'un gel | Temps de gel (min) | Durée de vie du gel (min) |
| 115 | 1,5 | 0,013 Thermolysine | Oui | 46 | 404 |
| 14 | 1,5 | 0,11 Trypsine | Oui | 48 | > 1500 |
| 3750 | 1,5 | 0,0004 collagénase) | Oui | 26 | 475 |

Les résultats montrent là encore que le modèle développé est généralisable à d'autres enzymes.

## Revendications

**1.** Procédé de préparation d'un biomatériau comprenant :

- au moins un monomère capable de former des polymères, ou un mélange desdits monomères et de leurs polymères, ledit monomère est une protéine choisie dans le groupe comprenant la fibrine, la gliadine, la myosine, la globuline (7S et HS), l'actine, la myoglobine, le collagène et ses dérivés, les protéines du petit lait, les protéines du soja et du blé, et les protéines du jaune et du blanc d'oeuf et/ou un saccharide capable de former des polymères choisi dans le groupe comprenant les carraghénanes, les alginates, les pectines, le chitosan, la cellulose, la chitine, le glycogène et l'amidon-un premier type d'enzyme capable de dégrader lesdits polymères, ledit premier type d'enzyme capable de dégrader lesdits polymères étant choisi, en fonction de la nature protéique ou saccharidique desdits polymères, dans le groupe comprenant les enzymes de la famille des métalloprotéinases, la famille des serines protéases, la famille des cystéines et aspartate protéases, et la famille ADAM, les carraghénases, les pectines lyases, les polygalacturonases, les pectines estérases, les alginates lyases et les phosphorylase
et
- éventuellement un second type d'enzyme capable d'effectuer des liaisons covalentes entre lesdits monomères,

ledit biomatériau se présentant soit sous la forme d'un gel, soit sous la forme d'une solution et étant susceptible d'effectuer successivement une transition solution/gel éventuellement sous l'action du second type d'enzyme puis, sous l'action du premier type d'enzyme, une seconde transition gel/solution,
ledit procédé étant **caractérisé en ce qu'**il comprend le mélange dans un solvant approprié: (i) d'au moins un monomère capable de former des polymères; (ii) d'un premier type d'enzyme capable de dégrader lesdits polymères; et éventuellement (iii) d'un second type d'enzyme capable d'effectuer des liaisons covalentes entre lesdits monomères ;
dans lequel mélange la concentration en monomères capables de former des polymères, la concentration en enzyme capable de dégrader lesdits polymères et éventuellement la concentration en enzyme capable d'effectuer des liaisons covalentes entre lesdits monomères sont choisies de sorte que la résolution des équations (I), (II), (III) et (IV) suivantes :

$$\frac{dg}{dt} = -\frac{V_P}{K_P + g} \times g + \frac{V_T}{K_T + s} \times s + V_H \quad (I)$$

$$\frac{ds}{dt} = \frac{V_P}{K_P + g} \times g - \frac{V_T}{K_T + s} \times s - \frac{V'_P}{K_P + s} \times s - V_H \quad (II)$$

$$\frac{df}{dt} = \frac{V'_P}{K_P + s} \times s \quad (III)$$

$$g_t + s_t + f_t = S_0 \quad (IV)$$

lesquelles équations décrivent respectivement l'évolution du nombre de monomères liés (dg) (I), l'évolution du nombre de monomères en solution (ds) (II) et l'évolution du nombre de monomères dégradés (df) en fonction du temps (dt) (III), et l'équation de conservation de masse (IV), où :

- g correspond à la quantité de monomères sous forme liée,
- t correspond au temps,
- $V_P$ correspond à la vitesse de l'enzyme capable de dégrader les polymères exprimée en quantité de monomères liés ramenés sous leur forme libre par ladite enzyme et par unité de temps,
- $K_P$ représente la constante de Michaelis de l'enzyme capable de dégrader les polymères,
- s représente la quantité de monomères sous forme libre,
- $V_T$ correspond à la vitesse de l'enzyme capable d'effectuer des liaisons covalentes entre les monomères exprimée en quantité de monomères sous forme libre liés par ladite enzyme et par unité de temps,
- $K_T$ représente la constante de Michaelis de l'enzyme capable d'effectuer des liaisons covalentes entre les monomères,
- $V_H$ représente la vitesse de liaison, par des liaisons de faible énergie et par unité de temps, de monomères sous forme libre, dans le cas de monomères capables de polymériser par de telles liaisons,
- $V'_P$ correspond à la vitesse de l'enzyme capable de dégrader les monomères sous forme libre exprimée en quantité de monomères dégradés, lesquels ne peuvent plus polymériser, générés par ladite enzyme et par unité de temps,
- $g_t$ correspond à la quantité de monomères sous forme liée au temps t,
- $s_t$ correspond à la quantité de monomères sous forme libre au temps t,
- $f_t$ correspond à la quantité de monomères dégradés qui ne sont plus capables de former des polymères au temps t,
- $S_0$ correspond à la quantité initiale de monomères,

permet d'obtenir un temps de gel et la durée de gel souhaités.

2. Procédé selon la revendication 1, dans lequel le solvant approprié est un solvant aqueux.

3. Procédé selon l'une quelconque des revendications 1 à 2, dans lequel la quantité de monomères capables de former des polymères est comprise entre 0,1 et 30% en poids par rapport au poids total du biomatériau.

4. Procédé selon l'une quelconque des revendications 1 à 3, dans lequel le rapport de la concentration en enzyme capable d'effectuer des liaisons covalentes entre les monomères sur la concentration d'enzymes capables de dégrader les polymères est supérieur ou égal à 1.

5. Procédé selon l'une quelconque des revendications 1 à 4, dans lequel la concentration en enzyme capable d'effectuer des liaisons covalentes entre les monomères est supérieure à 0,001 U/ml.

**6.** Procédé selon l'une quelconque des revendications 1 à 5, comprenant en outre une étape de lyophilisation du biomatériau sous forme de gel.

**7.** Biomatériau obtenu par un procédé selon l'une quelconque des revendications 1 à 6.

**8.** Biomatériau selon la revendication 7, dans lequel les deux types d'enzymes sont présents simultanément sous une forme non-inactivée.

**9.** Biomatériau selon la revendication 8, dans lequel l'enzyme est une métalloprotéinase.

**10.** Biomatériau selon la revendication 9, dans lequel l'enzyme est une thermolysine bactérienne.

**11.** Biomatériau selon l'une quelconque des revendications 7 à 10, dans lequel ledit monomère est une protéine capable de former des polymères, et le second type d'enzyme capable d'effectuer des liaisons covalentes entre les monomères de protéines est choisi dans le groupe comprenant la famille des lysyl oxydases et la famille des transglutaminases.

**12.** Biomatériau selon l'une quelconque des revendications 7 à 11, dans lequel le rapport de la concentration du second type d'enzyme sur la concentration du premier type d'enzyme est supérieur ou égal à 1.

**13.** Biomatériau selon l'une quelconque des revendications 7 à 12, comprenant en outre un solvant aqueux.

**14.** Biomatériau selon l'une quelconque des revendications 7 à 13, se trouvant sous la forme d'un gel lyophilisé.

## Claims

**1.** Method for preparing a biomaterial comprising :

- At least one monomer able to form polymers, or a mix of said monomers and their polymers, said monomer is a protein selected from the group comprising fibrin, gliadin, myosin, globulin (7S and HS), actin, myoglobin collagene and its derivates, whey proteins, soybean and wheat proteins, egg white and yolk proteins and/or a saccharide able to form polymers selected from the group comprising carraghenanes, alginates, pectines, chitosan, cellulose, chitine, glycogene and starch
- a first type of enzyme able to degrade said polymers, said first type of enzyme able to degrade said polymers is being selected, according to the proteic or saccharidic nature of said polymers, from the group comprising enzymes of the metalloproteinase family, serine protease family, cystein and aspartate protease family, ADAM family, carraghenases, pectine lyases, polygalacturonases, pectine esterases, alginate lyases and phosphorylases

and

- possibly a second type of enzyme able to make covalent bounds in between said monomers,

said biomaterial is presented in a gel form, or in a solution form and is able to make successively the solution/gel transition possibly thanks to the action of the second type of enzyme then, the action of the first type of enzyme allows a second solution/gel transition,

said method being **characterized in that** the mix is comprised in an appropriate solvent: (i) of at least one monomer able to form polymers ; (ii) a first type of enzyme able to degrade the said polymers ; and possibly (iii) a second type of enzyme able to form covalent bounds between said monomers ;

wherein mix the concentration of the monomers able to form polymers, the concentration of enzyme able to degrade said polymers and possibly the concentration of the second type of enzyme able to form covalent bounds between said monomers are chosen so as the solving of the following (I), (II), (III) and (IV) equations :

$$\frac{dg}{dt} = - \frac{V_p}{K_p + g} \times g + \frac{V_T}{K_T + s} \times s + V_g \qquad (I)$$

$$\frac{ds}{dt} = \frac{V_P}{K_P + g} \times g - \frac{V_T}{K_T + s} \times s - \frac{V'_P}{K_P + s} \times s - V_H \quad (II)$$

$$\frac{df}{dt} = \frac{V'_P}{K_P + s} \times s \quad (III)$$

$$g_t + s_t + f_t = s_0 \quad (IV)$$

which equations describe respectively the evolution of the number of bonded monomers (dg) (I), the evolution of the number of monomers in solution (ds) (II) and the evolution of the number of degraded monomers (df) according to time (dt) (III), and the equation of mass conservation (IV), where :

- g corresponds to the quantity of monomers in bonded form,
- t corresponds to time,
- $V_P$ corresponds to the speed of the enzyme able to degrade the polymers expressed in quantity of bonded monomers brought back under their free form by the said enzyme and by unit of time,
- $K_P$ represents the Michaelis constant of the enzyme able to degrade the polymers,
- s represents the quantity of monomers in free form,
- $V_T$ corresponds to the speed of the enzyme able to make covalent bonds in between the monomers expressed in quantity of free form monomers bonded by said enzyme and by time unit,
- $K_T$ represents the Michaelis constant of the enzyme able to make covalent bonds in between the monomers,
- $V_H$ represents the bounding speed, by low energy bonds and by time unit, of monomers in a free form, in the case of monomers able to polymerise by this kind of bonding,
- $V'_P$ corresponds to the speed of the enzyme able to degrade the monomers in free form expressed in quantity of monomers degraded, which can no more polymerise, generated by said enzyme and in time unit,
- $g_t$ corresponds the quantity of monomers in bonded form in time t,
- $s_t$ corresponds the quantity of monomers in free form in time t,
- $f_t$ corresponds the quantity of degraded monomers that are no more able to form polymers in time t,
- so corresponds the initial monomer quantity,

allows to obtain the desired gel time and useful gel life.

**2.** Method according to claim 1, wherein the appropriated solvent is an aqueous solvent.

**3.** Method according to any of claims 1 to 2, wherein the quantity of monomers able to form polymers in comprised between 0,1 and 30% in weight relative to the total weight of the biomaterial.

**4.** Method according to any of claims 1 to 3, wherein the ratio of the concentration of enzyme able to make covalent bonds in between the monomers on the concentration of enzymes able to degrade the polymers is above or equals to 1.

**5.** Method according to any of claims 1 to 4, wherein the concentration of enzyme able to make covalent bonds in between the monomers is above 0,001 U/ml.

**6.** Method according to any of claims 1 to 5, comprising besides a step of freeze-drying of the biomaterial in a gel form.

**7.** Biomaterial obtained by a method according to any of claims 1 to 6.

**8.** Biomaterial according to claim 7, wherein the two types of enzyme are simultaneously present in a non-inactivated form.

**9.** Biomaterial according to claim 8, wherein the enzyme is a metalloproteinase.

**10.** Biomaterial according to claim 9, wherein the enzyme is a bacterial thermolysine.

**11.** Biomaterial according to any of claims 7 to 10, wherein the said monomer is a protein able to form polymers, and the second type of enzyme able to make covalent bonds in between the protein monomers is selected from the group comprising the lysyl oxidase family and the transglutaminase family.

**12.** Biomaterial according to any of claims 7 to 11, wherein the ratio of the concentration of the second type enzyme on the concentration of the first type enzyme is above or equals to 1.

**13.** Biomaterial according to any of claims 7 to 12, comprising in addition an aqueous solvent.

**14.** Biomaterial according to any of claims 7 to 13, being in a freeze-dried gel form.

**Patentansprüche**

**1.** Zubereitungsverfahren eines Biomaterials, das Folgendes umfasst:

- wenigstens ein Monomer, das in der Lage ist, Polymere zu bilden, oder eine Mischung der genannten Monomere und ihrer Polymere, wobei das genannte Monomer ein Protein ist, das aus der Gruppe ausgewählt ist, das Fibrin, Gliadin, Myosin, Globulin (7S und HS), Aktin, Myoglobin, Kollagen und seine Derivate, die Proteine aus Buttermilch, die Proteine aus Soja und Weizen und die Proteine aus Eigelb- und Eiweiß und /oder ein Saccharid umfasst, das in der Lage ist, Polymere zu bilden, die aus der Gruppe ausgewählt sind, die Carraghene, Alginate, Pektine, Chitosan, Zellulose, Chitin, Glykogen und Stärke eines ersten Enzymtyps umfasst, der in der Lage ist , die genannten Polymere zu zersetzen, wobei der genannte erste Enzymtyp, der in der Lage ist, die genannten Polymere zu zersetzen, in Abhängigkeit von der Eiweiß- oder Saccharidart der genannten Polymere aus der Gruppe ausgewählt ist, die die Enzyme der Familie der Metalloproteinasen, der Familie der Serinprotasen, der Familie der Zysteine und der Aspartatproteasen und der Familie ADAM, die Carraghenasen, die Lyasepektine, die Polygalakturonasen, die Esterasenpektine, die Lyasenalginate und die Phosphrylasen umfassen und
- eventuell einen zweiten Enzymtyp, der in der Lage ist, die kovalenten Verbindungen zwischen den genannten Monomeren durchzuführen,

wobei das genannte Biomaterial entweder die Form eines Gels oder die Form einer Lösung aufweist und geeignet ist, sukzessive einen Übergang Lösung / Gel, eventuell unter der Wirkung des zweiten Enzymtyps durchzuführen, dann unter der Wirkung des ersten Enzymtyps einen zweiten Übergang Gel /Lösung, wobei das genannte Verfahren **dadurch gekennzeichnet ist, dass** es die Mischung in einer geeigneten Lösung umfasst: (i) von wenigstens einem Monomer, das in der Lage ist, Polymere zu bilden; (ii) von einem ersten Enzymtyp, der in der Lage ist, die genannten Polymere zu zersetzen; und eventuell (i-ii) einem zweiten Enzymtyp, der in der Lage ist, kovalente Verbindungen zwischen den genannten Monomeren durchzuführen; wobei in der genannten Mischung die Konzentration an Monomeren, die in der Lage sind, Polymere zu bilden, die Konzentration an Enzymen, die in der Lage sind, die genannten Polymere zu zersetzen und eventuell die Konzentration an Enzymen, die in der Lage sind, kovalente Verbindungen zwischen den genannten Monomeren herzustellen, derart ausgewählt sind, dass die Auflösung der folgenden Gleichungen (I), (II), (III) und (IV):

$$\frac{dg}{dt} = -\frac{V_p}{K_p + g} \times g + \frac{V_r}{K_r + s} \times s + V_s \qquad (I)$$

$$\frac{ds}{dt} = \frac{V_p}{K_p + g} \times g - \frac{V_r}{K_r + s} \times s - \frac{V'_p}{K_p + s} \times s - V_s \qquad (II)$$

$$\frac{df}{dt} = \frac{V'_p}{K_p + s} \times s \qquad (III)$$

$$g_t + s_t + f_t = S_0 \qquad (IV)$$

ist,
wobei diese Gleichungen jeweils die Entwicklung der Zahl der verbundenen Monomere (dg) (I), die Entwicklung der Zahl der Monomere in einer Lösung (ds) (II) und die Entwicklung der Zahl der zersetzten Monomere (df) in Abhängigkeit von der Zeit (dt) (III) beschreibt und die Gleichung der Massekonservierung (IV), bei der:

- g der Menge der Monomere in gebundener Form entspricht,
- t der Zeit entspricht,
- $V_P$ der Geschwindigkeit des Enzyms entspricht, das in der Lage ist, die Polymere zu zersetzen, die als Menge der gebundenen, durch das genannte Enzym und durch die Zeiteinheit in ihre freie Form zurückgebrachten Monomere ausgedrückt werden,
- $K_P$ die Michaelis-Konstante des Enzyms darstellt, das zum Zersetzen der Polymere in der Lage ist,
- s die Monomermenge in freier Form darstellt,
- $V_r$ der Geschwindigkeit des Enzyms entspricht, das in der Lage ist, kovalente Verbindungen zwischen den Monomeren durchzuführen, die als Monomermenge in freier Form ausgedrückt wird, die durch das genannte Enzym und durch die Zeiteinheit verbunden sind, auszudrücken,
- $K_r$ die Michaeliskonstante des Enzyms darstellt, das in der Lage ist, kovalente Verbindungen zwischen den Monomeren durchzuführen,
- $V_R$ die Verbindungsgeschwindigkeit durch Verbindungen mit geringer Energie und durch die Zeiteinheit von Monomeren in freier Form in dem Fall von Monomeren darstellt, die in der Lage sind, durch derartige Verbindungen zu polymerisieren,
- $V'_P$ der Geschwindigkeit des Enzyms entspricht, das in der Lage ist, die Monomere in freier Form zu zersetzen, die in einer zersetzten Monomermenge ausgedrückt wird, welche nicht mehr polymerisieren können und durch das genannte Enzym durch die Zeiteinheit generiert werden,
- $g_t$ der Monomermenge in gebundener Form in der Zeit t entspricht,
- $s_t$ der Monomermenge in freier Form in der Zeit t entspricht,
- $f_t$ der Menge der zersetzten Monomere entspricht, die nicht mehr in der Lage sind, Polymere in der Zeit t zu bilden,
- $S_o$ der anfänglichen Monomermenge entspricht,

erlaubt den Erhalt einer gewünschten Gelzeit und der gewünschten Geldauer.

2. Verfahren gemäß Anspruch 1, in dem die geeignete Lösung eine wässrige Lösung ist.

3. Verfahren gemäß Anspruch 1 bis 2, in dem die Menge an Monomeren, die in der Lage sind, Polymere zu bilden, zwischen 0,1 und 30 Gew.-% im Verhältnis zum Gesamtgewicht des Biomaterials inbegriffen ist.

4. Verfahren gemäß Anspruch 1 bis 3, in dem das Verhältnis der Konzentration an Enzymen, die in der Lage sind, kovalente Verbindungen zwischen den Monomeren herzustellen, zur Konzentration an Enzymen, die in der Lage sind, die Polymere zu zersetzen, höher als oder gleich 1 ist.

5. Verfahren gemäß Anspruch 1 bis 4, in dem die Konzentration an Enzymen, die in der Lage sind, kovalente Verbindungen zwischen den Monomeren herzustellen, höher als 0,001 E/ml ist.

6. Verfahren gemäß Anspruch 1 bis 5, das darüber hinaus eine Gefriertrockungsstufe des Biomaterials in Gelform umfasst.

7. Biomaterial, das aus einem Verfahren gemäß Anspruch 1 bis 6 erhalten wird.

8. Biomaterial gemäß Anspruch 7, in dem die zwei Enzymtypen gleichzeitig in einer nicht aktivierten Form vorhanden

sind.

9. Biomaterial gemäß Anspruch 8, in dem das Enzym eine Metalloproteinase ist.

10. Biomaterial gemäß Anspruch 9, in dem das Enzym ein bakterielles Thermolysin ist.

11. Biomaterial gemäß Anspruch 7 bis 10, in dem das genannte Monomer ein Protein ist, das in der Lage ist, Polymere zu bilden, und der zweite Enzymtyp, der in der Lage ist, kovalente Verbindungen zwischen den Proteinmonomeren herzustellen, aus der Gruppe ausgewählt ist, die die Familie der Lysyloxidasen und die Familie der Transglutaminasen umfasst.

12. Biomaterial gemäß Anspruch 7 bis 11, in dem das Verhältnis der Konzentration des zweiten Enzymtyps zur Konzentration des ersten Enzymtyps höher als oder gleich 1 ist.

13. Biomaterial gemäß Anspruch 7 bis 12, das darüber hinaus eine wässrige Lösung umfasst.

14. Biomaterial gemäß Anspruch 7 bis 13, das sich in Form eines gefriergetrockneten Gels befindet.

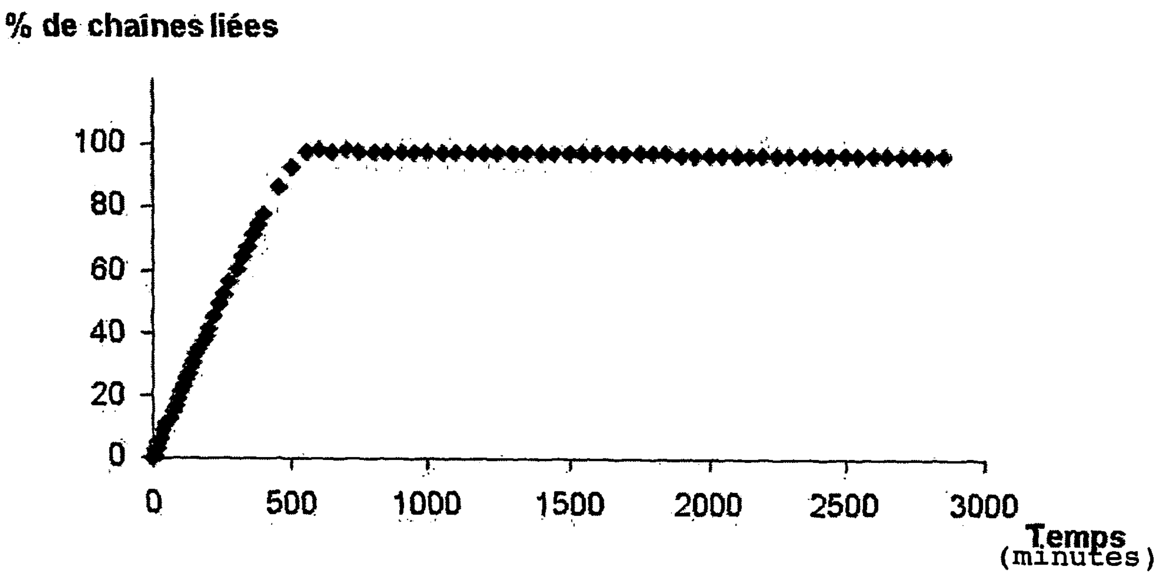
% de chaînes liées
100
80
60
40
20
0
0
500
1000
1500
2000
2500
3000
Temps
(minutes)

Figure 1

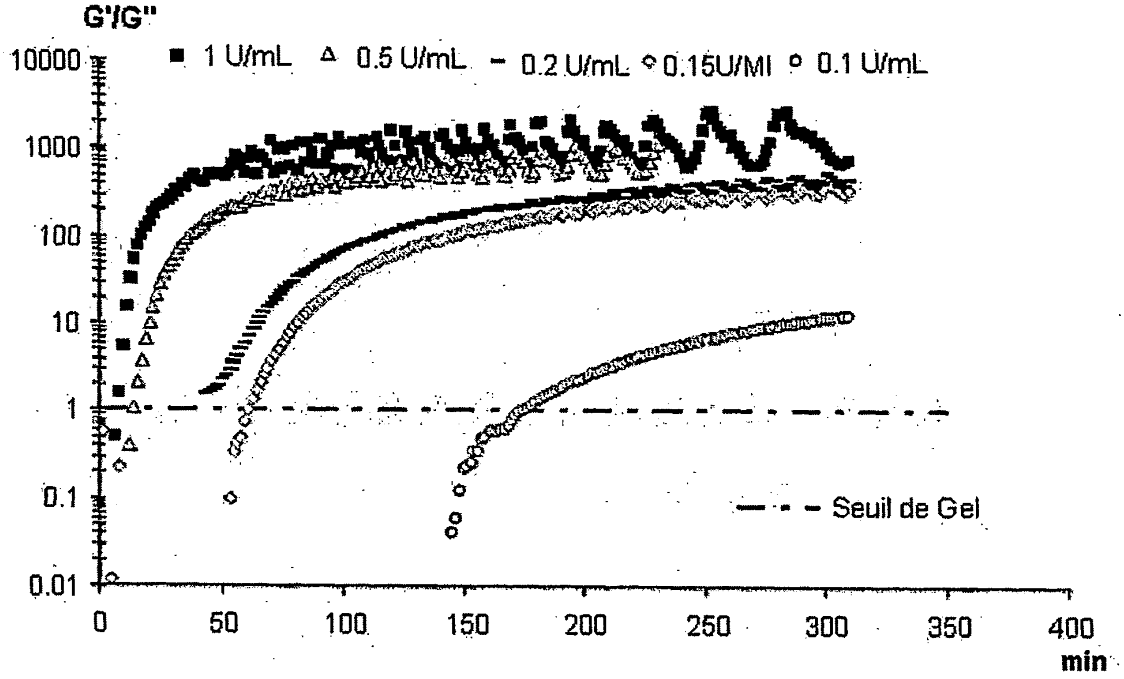
G'/G"
1 U/mL
0.5 U/mL
0.2 U/mL
0.15U/Ml
0.1 U/mL
10000
1000
100
10
1
0.1
0.01
0
50
100
150
200
250
300
350
400
min
Seuil de Gel

Figure 2

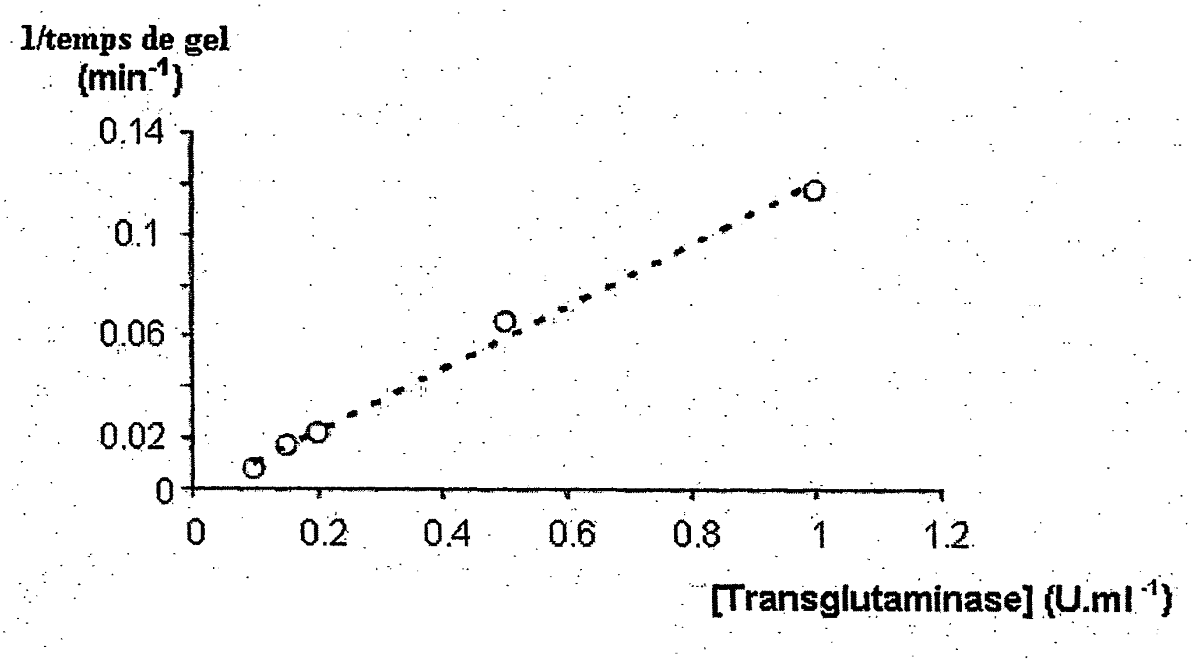
1/temps de gel
(min-1)
0.14
0.1
0.06
0.02
0
0
0.2
0.4
0.6
0.8
1
1.2
[Transglutaminase] (U.ml-1)

Figure 3

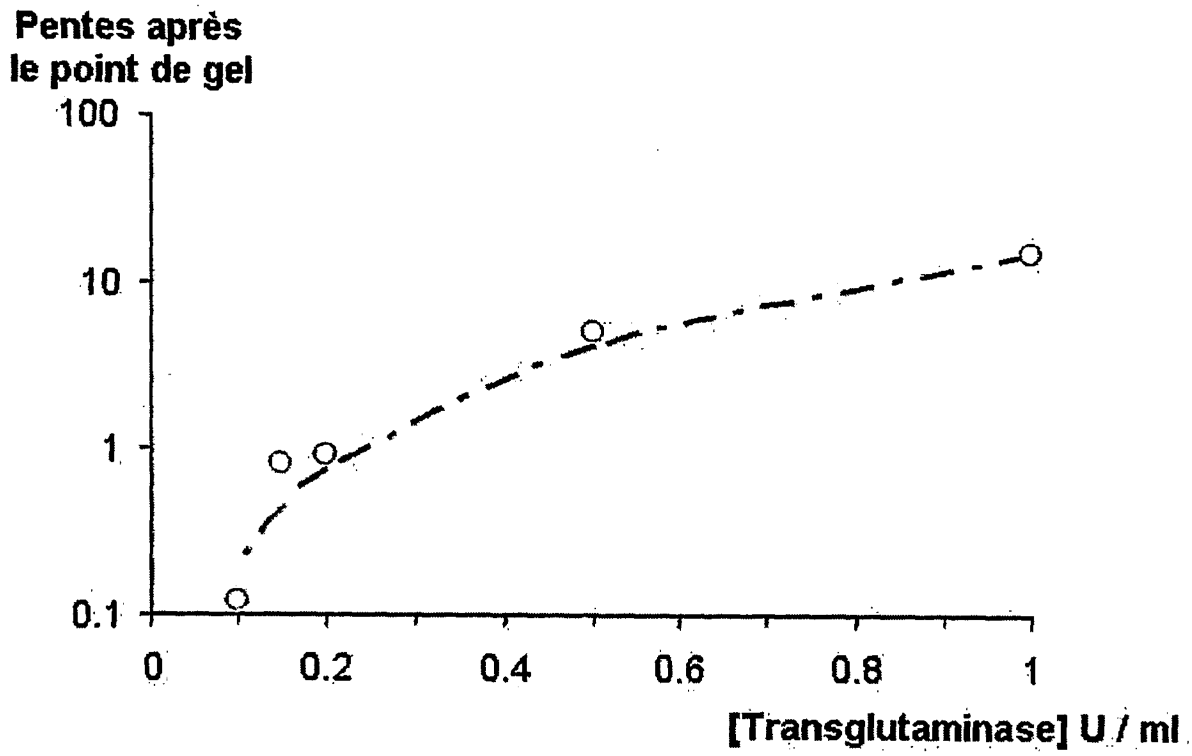
Pentes après
le point de gel
100
10
1
0.1
0
0.2
0.4
0.6
0.8
1
[Transglutaminase] U / ml

Figure 4

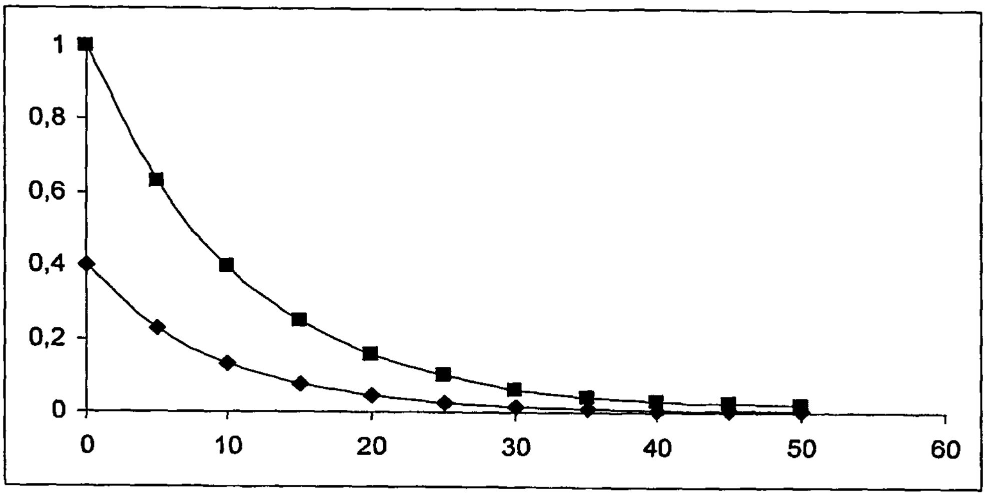


Fiqure 5

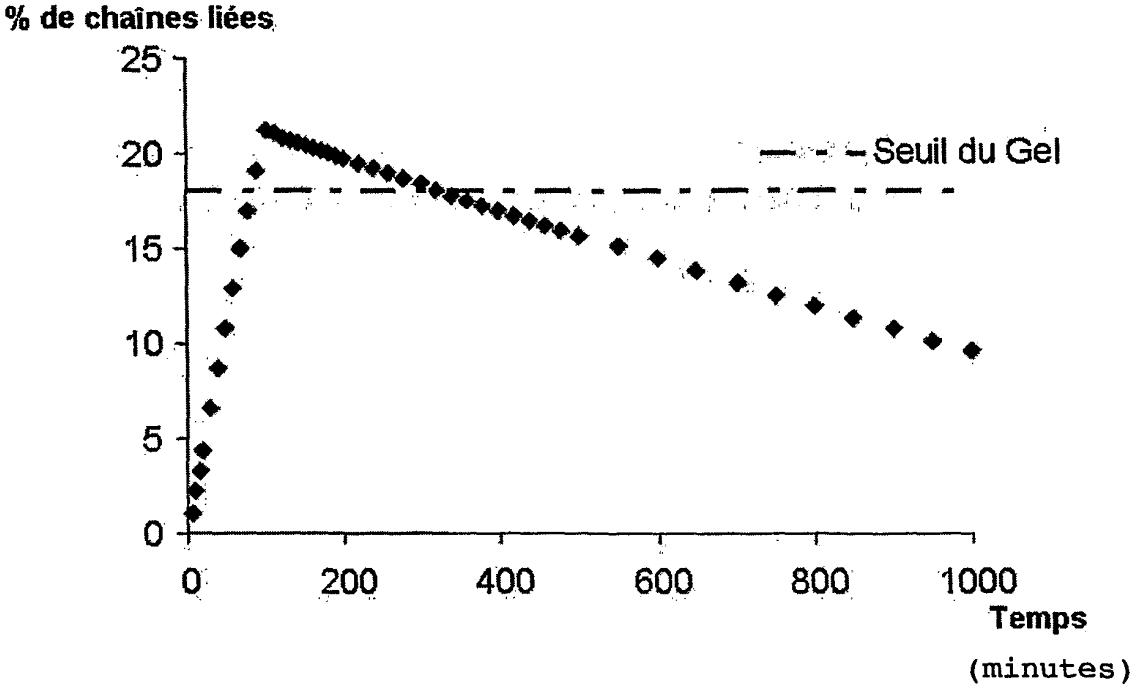
% de chaînes liées
25
20
15
10
5
0
0
200
400
600
800
1000
Seuil du Gel
Temps
(minutes)

Figure 6

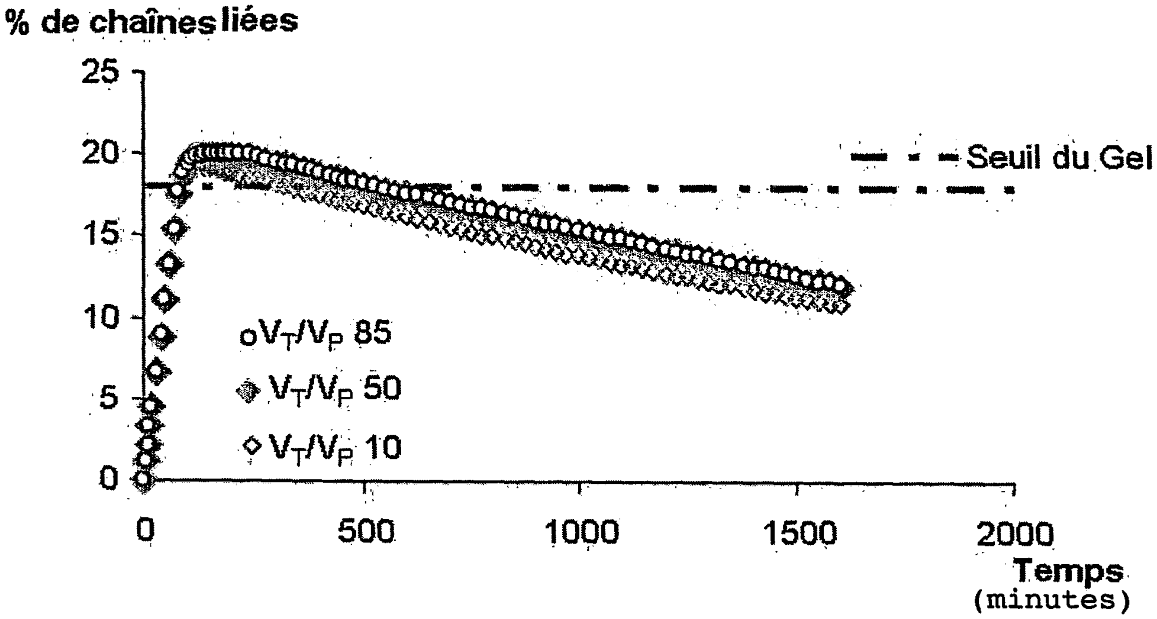
% de chaînes liées
25
20
15
10
5
0
0
500
1000
1500
2000
Temps
(minutes)
Seuil du Gel
$V_T/V_P$ 85
$V_T/V_P$ 50
$V_T/V_P$ 10

Figure 7

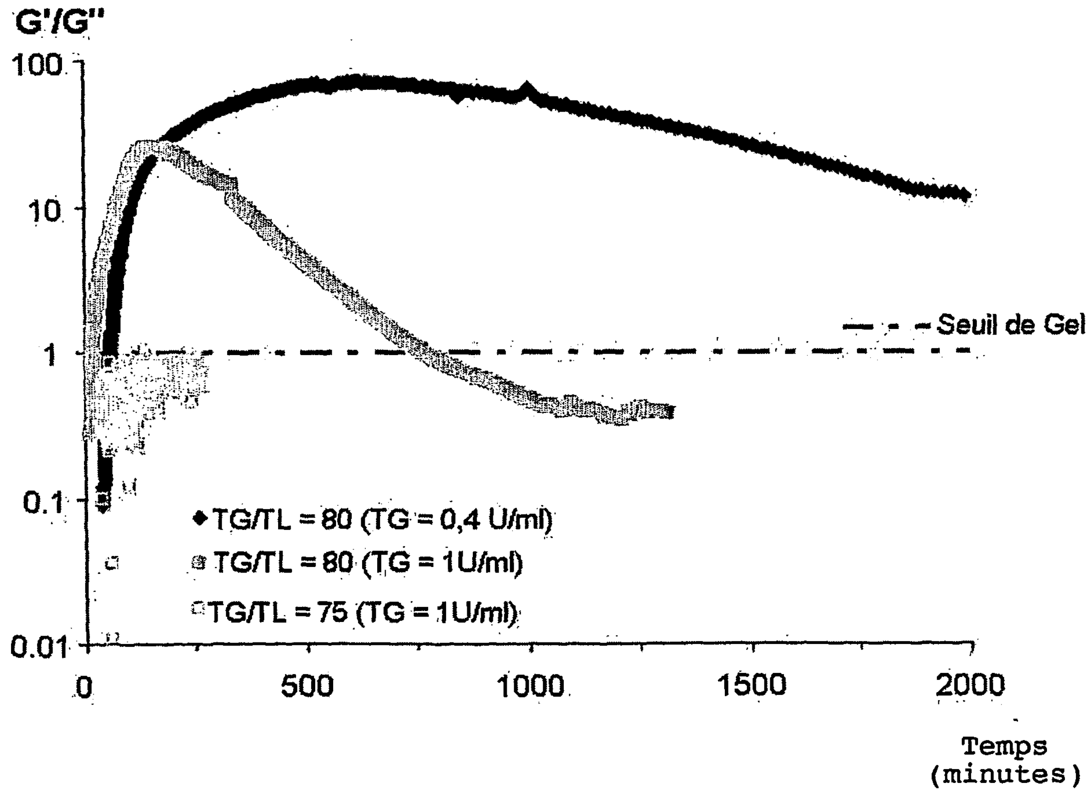
G'/G"
100
10
1
0.1
0.01
0
500
1000
1500
2000
Seuil de Gel
TG/TL = 80 (TG = 0,4 U/ml)
TG/TL = 80 (TG = 1U/ml)
TG/TL = 75 (TG = 1U/ml)
Temps
(minutes)

Figure 8

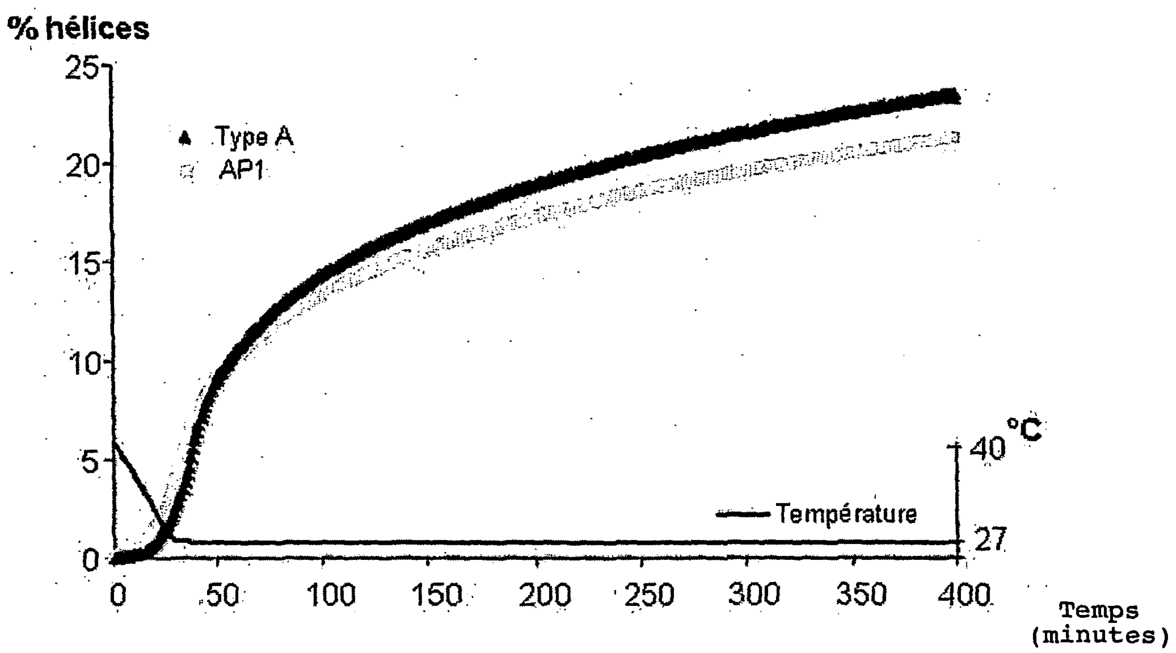
% hélices
25
20
15
10
5
0
Type A
AP1
Température
40
27
°C
0
50
100
150
200
250
300
350
400
Temps
(minutes)

Figure 9

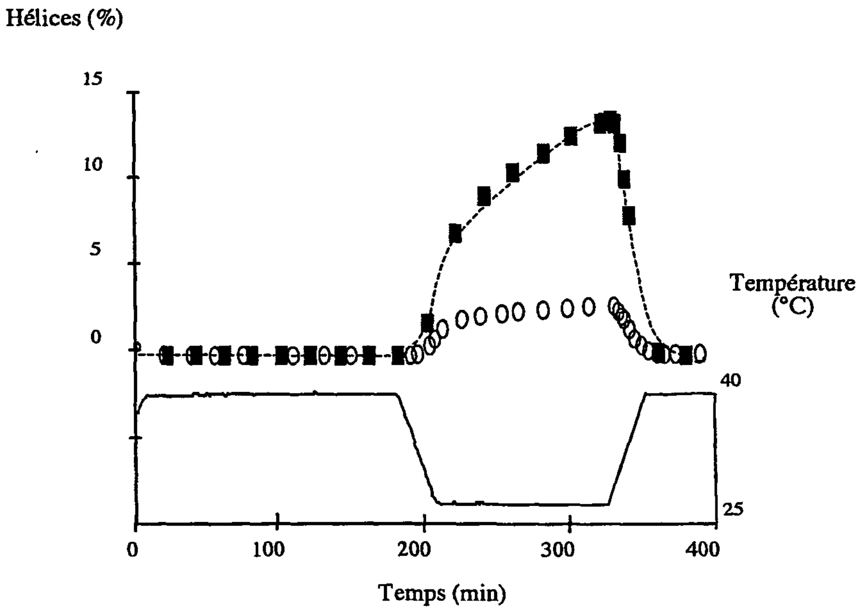
Hélices (%)
15
10
5
0
Température
(°C)
40
25
0
100
200
300
400
Temps (min)

Figure 10

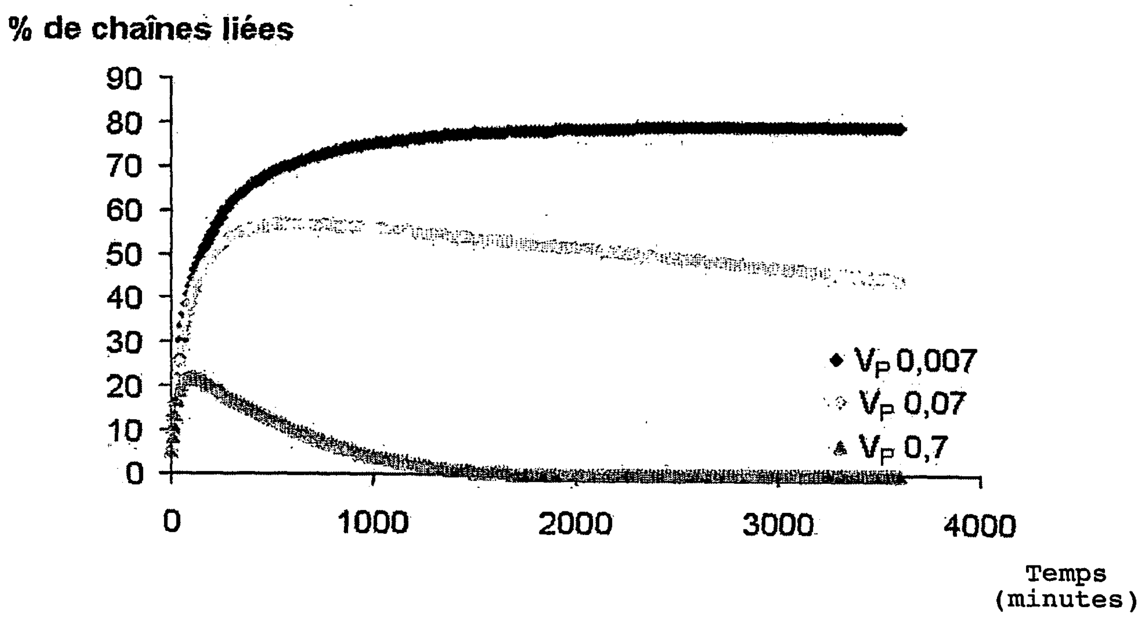
% de chaînes liées
90
80
70
60
50
40
30
20
10
0
0
1000
2000
3000
4000
Temps
(minutes)
$V_P$ 0,007
$V_P$ 0,07
$V_P$ 0,7

Figure 11

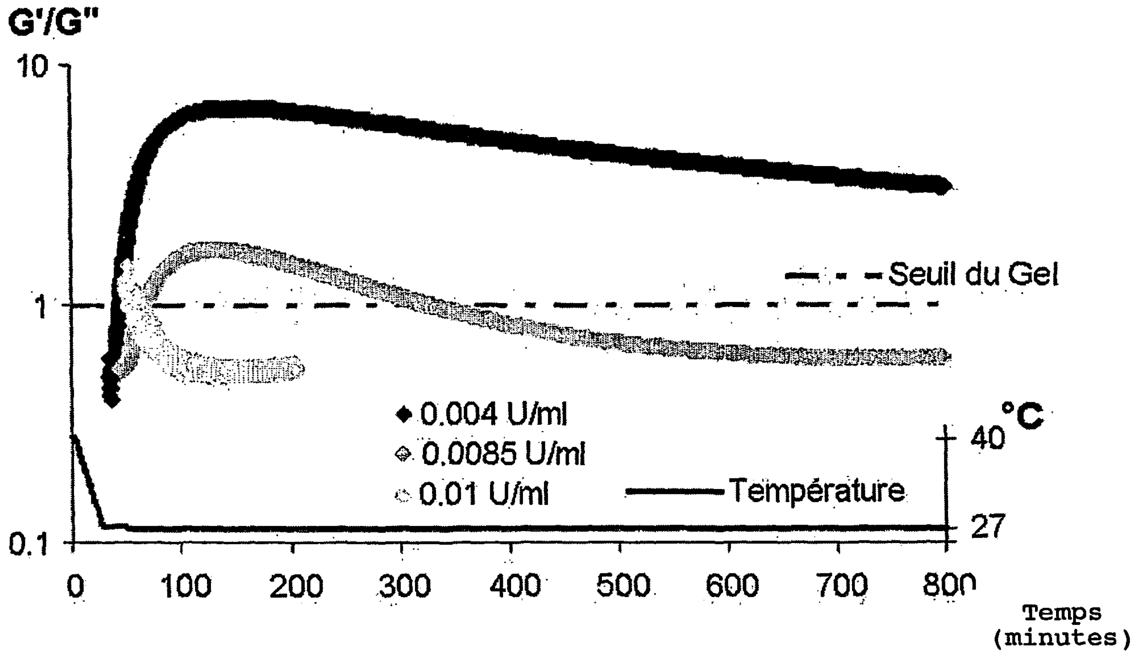
G'/G"
10
1
0.1
Seuil du Gel
0.004 U/ml
0.0085 U/ml
0.01 U/ml
Température
°C
40
27
0
100
200
300
400
500
600
700
800
Temps
(minutes)

Figure 12

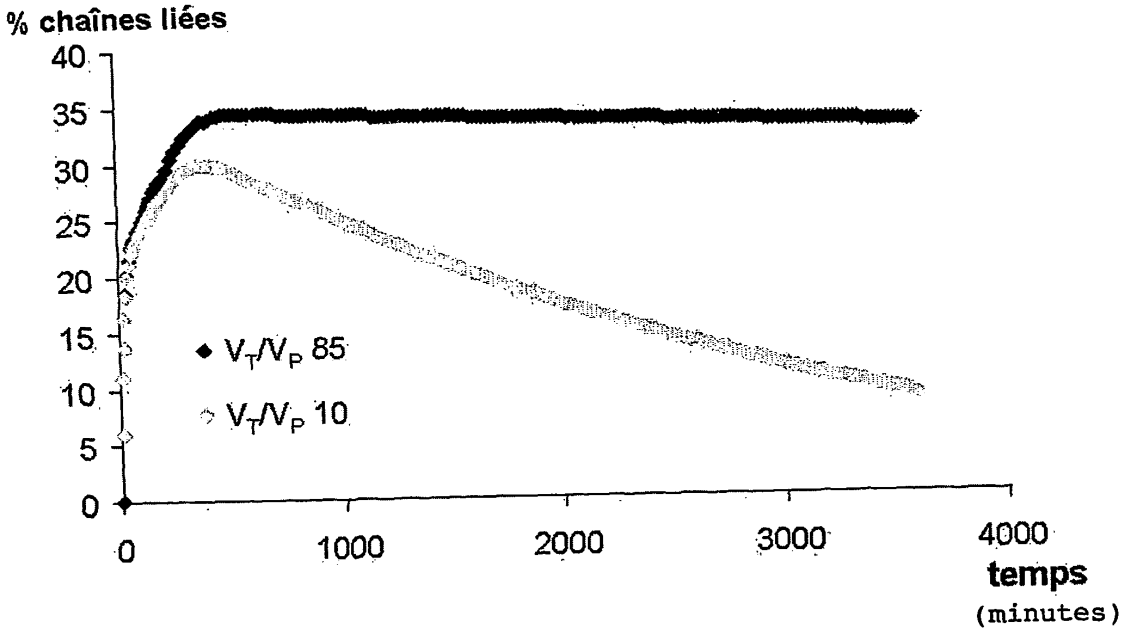
% chaînes liées
40
35
30
25
20
15
10
5
0
0
1000
2000
3000
4000
temps
(minutes)
◆ $V_T/V_P$ 85
◇ $V_T/V_P$ 10

Figure 13

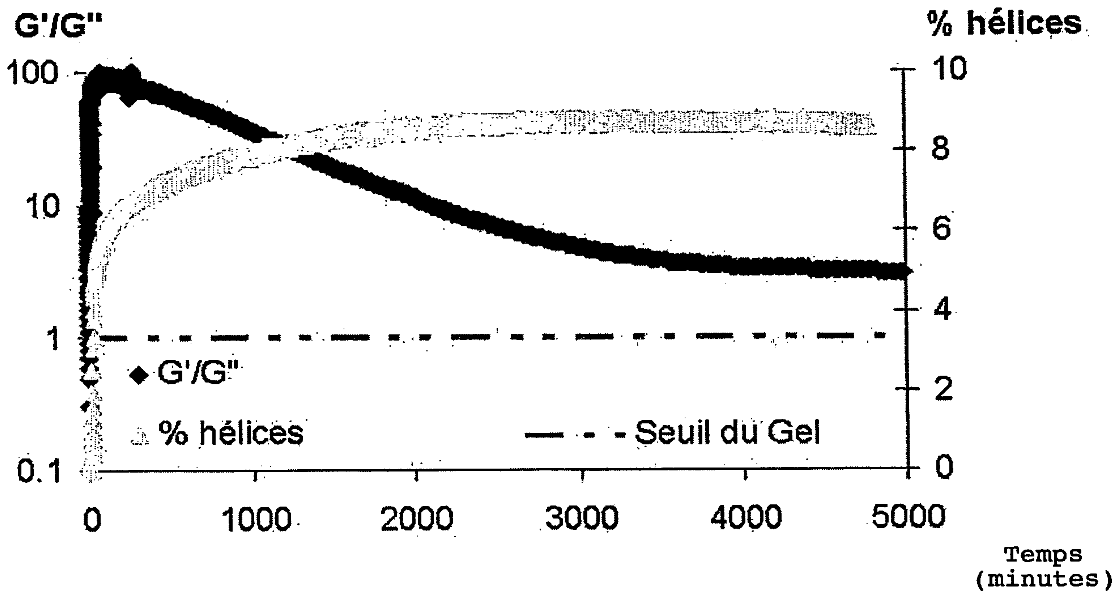
G'/G"
% hélices
100
10
1
0.1
10
8
6
4
2
0
0
1000
2000
3000
4000
5000
◆ G'/G"
% hélices
Seuil du Gel
Temps (minutes)

Fiqure 14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **PRICE ; STEVEN.** Fundamentals of Enzymology: The Cell and Molecular Biology of Catalytic Proteins. Oxford University Press
- **SEGEL.** Classics Library, Enzyme Kinetics. Wiley, 1993
- **MATSUBARA et al.** *Biochem. Biophys. Res. Commun.,* 1965, vol. 21, 242-247
- **LIGNE et al.** *Biochim. Biophys. Acta.,* 1997, vol. 1337, 143-148
- **BRONSON.** Equations Différentielles. 1994
- **FUCHSBAUER et al.** *Biomaterials,* 1999, vol. 17, 1481-1488
- **FADDA et al.** *Biophys. J.,* 2003, vol. 85 (5), 2808-2817
- **BERRY et al.** *Biochim. Biophys. Acta,* 2000, vol. 1524, 110-117
- **GIRAUDIER et al.** *Biomacromolecules,* 2004, vol. 5 (5), 1662-1666
- **PERSIKOV et al.** *Biochemistry,* 2000, vol. 39, 14960-14967
- **PEZRON.** Physical Networks. Elsevier Applied Science, 1990
- **JOLY-DUHAMEL et al.** *Langmuir,* 2002, vol. 18, 7158-7166
- **JOLY-DUHAMEL et al.** *Langmuir,* 2002, vol. 18, 7208-7217